# EUROPEAN PATENT APPLICATION

(11) **EP 4 170 022 A1**
(43) Date of publication of application: **26.04.2023**
(21) Application number: 21382940.1
(22) Date of filing: 19.10.2021
(51) Int. Cl.: C12N 7/00

(54) **RECOMBINANT AFRICAN SWINE FEVER VIRUS AND USES THEREOF**

(71) Applicant: Consejo Superior De Investigaciones Científicas, 28006 Madrid (ES); Icahn School of Medicine at Mount Sinai, New York, NY 10029 (US)
(72) Inventor: REVILLA NOVELLA, Yolanda, E - 28006 Madrid (ES); PÉREZ NÚÑEZ, Daniel, E - 28006 Madrid (ES); GARCÍA SASTRE, Adolfo, New York, 10029 (US)
(74) Representative: ABG Intellectual Property Law, S.L.

(57) **Abstract**

The present invention relates a recombinant African swine fever virus (ASFV), characterized in that the genome of the recombinant ASFV comprises at least one miRNA target sequence, the miRNA target sequence is found within a gene that is essential for viral replication and the miRNA target sequence is specific for a miRNA which is expressed in a viral target cell.

## Description

### FIELD OF THE INVENTION

The present invention relates to the field of recombinant virus technology, in particular, recombinant African swine fever virus (ASFV) and the use of recombinant ASFV in the prevention and/or treatment of diseases caused by infection by ASFV as well as vaccine or immunogenic compositions comprising recombinant ASFV.

### BACKGROUND OF THE INVENTION

African swine fever virus (ASFV), the only member of the family Asfarviridae, is a large, complex double-stranded DNA virus that is highly infectious and responsible for a serious disease in wild boar and domestic pigs. ASFV targets porcine alveolar macrophages, which allow ASFV cytoplasmic replication *in vivo* and virus spread.

ASFV is the etiological agent of African swine fever, which lastly emerged from East Africa to the Caucasus in 2007, where it spread to affect 28 countries in Europe, Oceania and Asia, including China. The virus is now endemic in China and is currently affecting neighboring countries such as Vietnam, Laos, Myanmar, Korea and the Philippines. Millions of domestic pigs have been sacrificed in Asia in 2020, and the virus has extended through Poland, Hungary and Baltic countries, representing a menace for several other European countries such as Germany and Belgium.

The situation is economically dramatic, unbalancing the food chain. ASFV is currently becoming a global threat and the lack of efficient vaccines against ASFV raises serious industrial and ecological concerns worldwide. The rapid spread of the disease proved eradication and regionalization measures to be insufficient for control of the current epidemic. Thus, there is a need for new approaches for epidemic control directed to the development of effective vaccines against African swine fever virus.

### SUMMARY OF THE INVENTION

The authors of the present invention have found that the insertion of at least one microRNA (miRNA) target site or sequence into the ASFV genome within a gene that is essential for viral replication, results in ASFV unable of replicating in viral target cells which express miRNA specific for the at least one miRNA target sequence inserted in the viral genome, in turn, allowing the development of effective vaccines against ASFV.

Therefore, in a first aspect the invention relates to a recombinant African swine fever virus (ASFV), characterized in that
(i) the genome of the recombinant ASFV comprises at least one miRNA target sequence,
(ii) the miRNA target sequence is found within a gene that is essential for viral replication and
(iii) the miRNA target sequence is specific for a miRNA which is expressed in a viral target cell.

In a second aspect, the present invention relates to a polynucleotide comprising a first, second and third regions, wherein the first region comprises the region of an ASFV gene which encodes the 3'-UTR of the mRNA encoded by said gene, which region is modified by replacing at least part of said region by at least one miRNA target site, wherein the first region is flanked by the second and third regions, and wherein said second and third regions are the ASFV genomic regions which naturally flank the first genomic region in the ASFV genome.

In a third aspect, the invention relates to a vector comprising the polynucleotide according to the invention.

In a fourth aspect, the invention relates to a host cell comprising the vector according to the invention.

In a fifth aspect, the invention relates to an immunogenic composition or a vaccine composition comprising the recombinant ASFV according to the invention, a polynucleotide according to the invention or a vector according to the invention and a pharmaceutically suitable carrier or excipient.

In a sixth aspect, the invention relates to a recombinant ASFV according to the invention, a polynucleotide according to the invention, a vector according to the invention or a vaccine or immunogenic composition according to the invention, for use in the prevention or treatment of a disease caused by the infection by ASFV.

In a seventh aspect, the invention relates to a method of inducing a protective immune response to an ASFV infection in an animal, said method comprising administering to said animal a recombinant ASFV according to the invention, a polynucleotide according to the invention, a vector according to the invention or a vaccine composition according to the invention.

In an eighth aspect, the invention relates to a method for producing a recombinant African swine fever virus (ASFV), the method comprising:
(i) modifying permissive viral target cells by
   - introducing a polynucleotide comprising a first, second and third regions, wherein the first region comprises the region of an ASFV gene essential for viral replication which encodes the 3'-UTR of the mRNA encoded by said gene, which region is modified by replacing at least part of said region by at least one miRNA target site, wherein the first region is flanked by the second and third regions and wherein said second and third regions are the ASFV genomic regions which naturally flank the first genomic region in the ASFV genome, wherein the polynucleotide further comprises a reporter gene between the second and third region,
   - infecting the cells with an ASFV strain in which the gene that is essential for viral replication does not contain a miRNA target site, and
   - introducing means capable of creating a double strand DNA break in the genome of said ASFV strain within or at the vicinity of the region encoding the 3'UTR of mRNA encoded by the essential gene for viral replication,
(ii) maintaining the cells under conditions adequate for the double-strand DNA break in the ASFV genome to take place and to allow homologous recombination between the ASFV genome containing the DNA break and the second and third regions of the polynucleotide, thereby resulting in the replacement of the region encoding the 3'UTR of mRNA encoded by the ASFV gene essential for viral replication by the first region of the polynucleotide and the reporter gene, and
(iii) recovering the recombinant ASFV from the supernatant and/or from the whole cell extract and selecting the ASFV virions which contain the reporter gene.

### BRIEF DESCRIPTION OF THE FIGURES

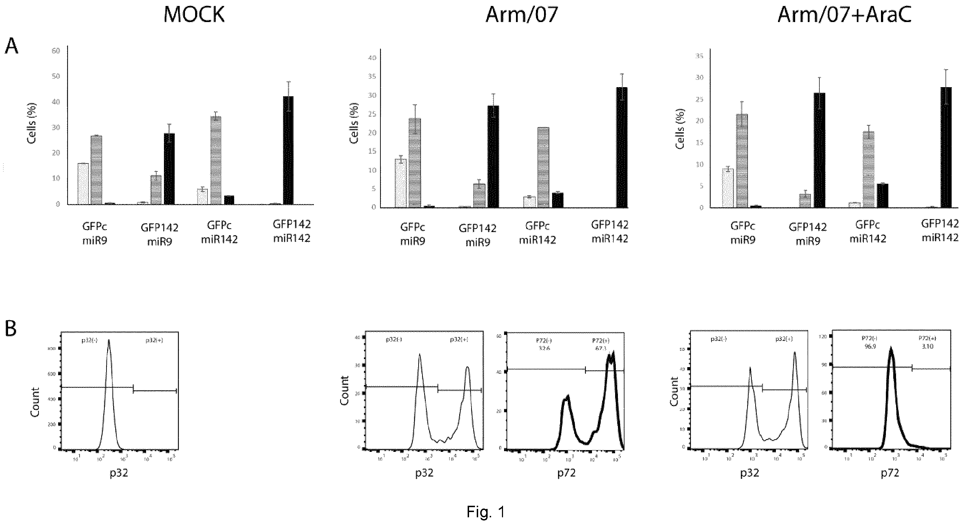

**Figure 1****.** COS cells were transfected with the following combinations: EGFPc+miR9, EGFP_142+miR9, EGFPc+miR142, EGFP_142+miR142 (1µg:3µg). 24hpt cells were mock-infected or infected with Arm/07 strain (MOI=0.2). 24h after, cells were stained with anti-p32 (early ASFV protein) and analyzed by FACS. Control cells were stained with anti-p72 (late ASFV protein). Percentage of cells in Q1 (GFP+/DsRed- (gray bars), Q2 (GFP+/DsRed+) (lined bars) and Q3 (GFP-/DsRed+) (solid bars) boxes is shown in (A), referred to total or only transfected cells. Infected cells is shown in (B), expression both early and late p32 and p72 proteins (-AraC) or only expressing early p32 protein (+AraC).

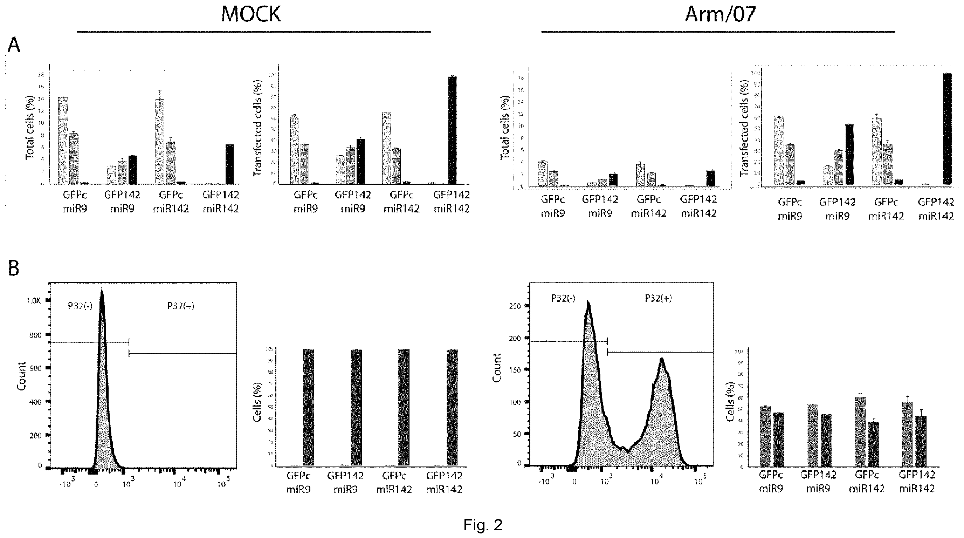

**Figure 2****.** COS cells were MOCK infected or infected with Arm/07 strain (MOI=0.2). After adsorption, cells were transfected with the GFP/miRNA-DsRed combination described (1µg:3µg) and 16h after cells were stained with anti-p32 (ASFV protein) and analyzed by FACS. The percentage of cells in Q1 (gray bars), Q2 (lined bars) and Q3 (black bars) is shown in (A), referred to total or only transfected cells. Finally, percentage of infected cells is shown in (B), where infected cells were positively strained for p32 (gray bars) while non-infected cells were negatively stained for p32 (black bars).

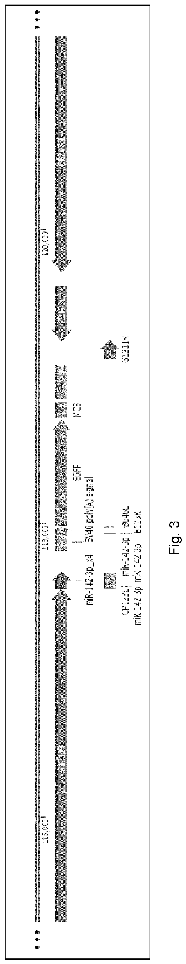

**Figure 3****.** Genetic map of the Arm07/CBM/c2 indicating the miR142 target sequences and the EGFP gene under p72 promoter, downstream of ASFV G1211R gene (ASFV DNA polymerase).

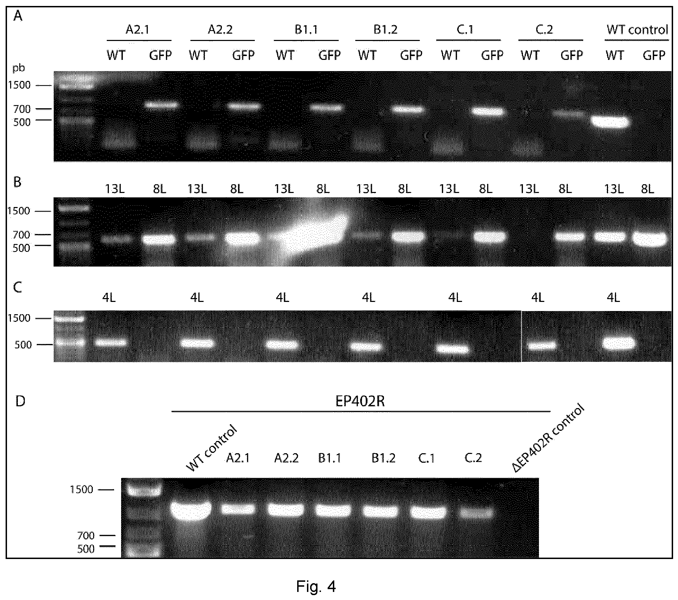

**Figure 4****.** PCR for the amplification of specific sequence for (A) Arm-WT or recombinant G12114-miR142 or the presence of the genes (B) MGF110-13L (13L), MGF360-8L (8L), (C) MGF360-4L (4) or (D) EP402R. Armc2-WT was used as WT control and Arm-ΔEP402R as a negative control for EP402R amplification. Water was used as negative control and no amplification was found with any gene (not shown).

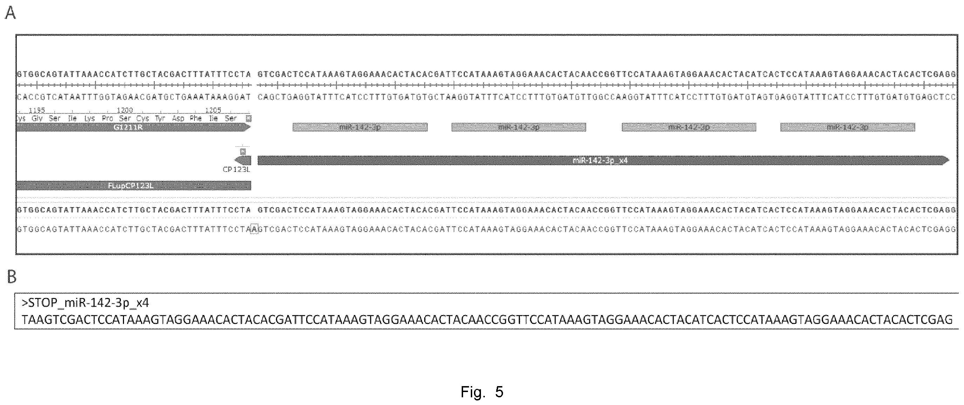

Figure 5(A) Sequence of the PCR product of the region amplified by the oligos G12114-Fw and 5'GFP-Rv as obtained by Sanger sequencing. Compared to the expected sequence, only an indel downstream the STOP codon and upstream the tandem target sequences of miR142 was found (indicated as boxes containing an X). (B) Sequence of the "STOP_miR142-3p_x4" which comprise four copies in tandem of the miR142-3p target sequence.

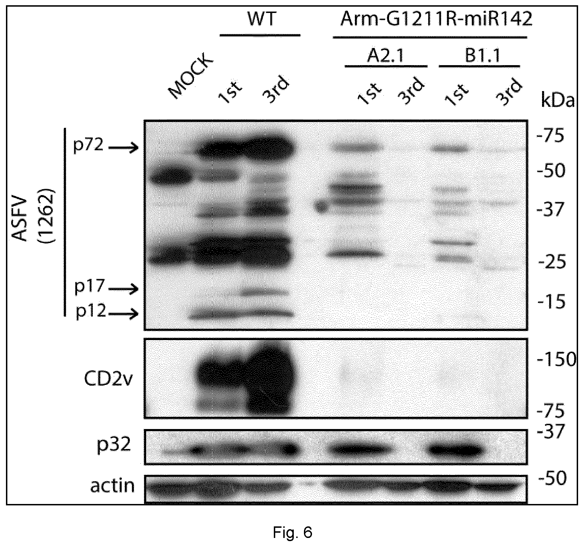

**Figure 6****.** Porcine alveolar macrophages (PAM) were mock infected or infected with Arm07/CBM/c2 WT or G1211R-miR142-GFP viruses (MOI= 0.005) for 72h (1st passage) or from virus obtained of successive passages as explained in Fig. 5. Cells from first (1st) and third (3rd) passages were lysed in RIPA buffer and lysates were separated by 7 to 20% SDS-PAGE, followed by immunoblotting with anti-ASFV 1262 (total viral ASFV proteins), anti-CD2v, anti-p32 and anti-actin antibodies.

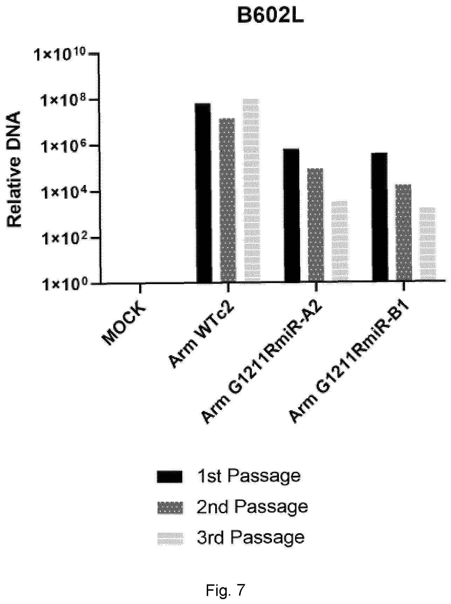

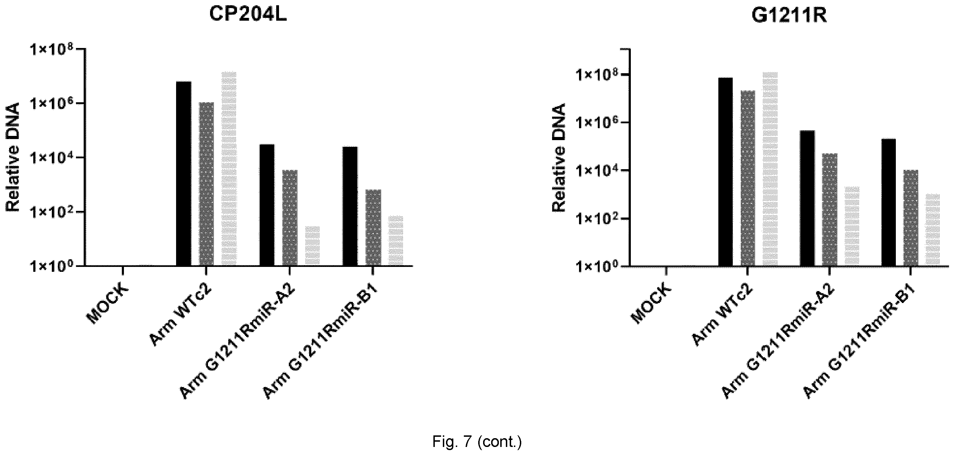

**Figure 7****.** Viral DNA detection in porcine alveolar macrophages infected by Arm07/CBM/c2 WT or G1211R-miR142-GFP as indicated in Fig.5. Porcine alveolar macrophages were mock infected or infected with Arm07/CBM/c2 WT or G1211R-miR142-GFP over three passages. Cells were collected as indicated and total DNA extracted. qPCR analyses of viral DNA from amplifying B602L, CP204L or G1211R were performed.

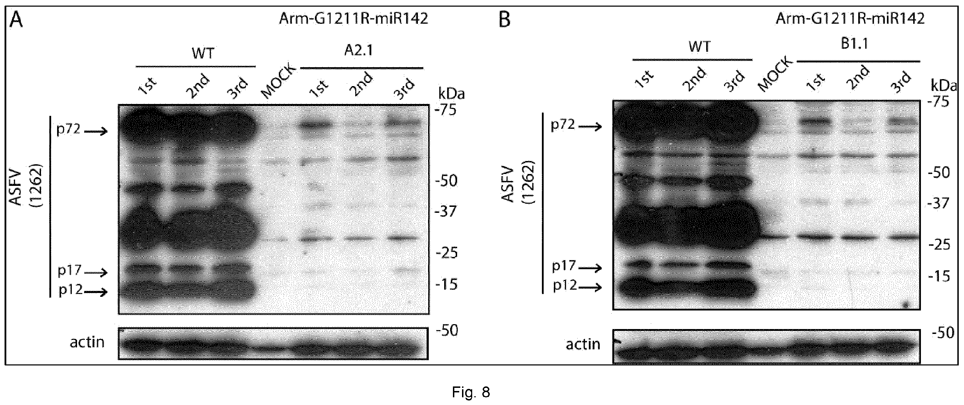

**Figure 8****.** COS cells were mock infected or infected with Arm07/CBM/c2 WT or G1211R-miR142-GFP viruses from passages in PAM (1st, 2nd and 3rd) as indicated in Fig. 5 for 72hpi. Cells were lysed in RIPA buffer and lysates were separated by 7 to 20% SDS-PAGE, followed by immunoblotting with anti-ASFV 1262 (total viral ASFV proteins) and anti-actin antibodies.

### DETAILED DESCRIPTION OF THE INVENTION

The present invention relates to recombinant African swine fever virus (ASFV) and polynucleotides comprising the genome of the recombinant ASFV as well as to the prevention or treatment of diseases associated with ASFV by means of vaccines and immunogenic compositions comprising said recombinant ASFV.

### Recombinant African swine fever virus (ASFV) according to the invention

The authors of the present invention have demonstrated that, surprisingly, ASFV, another large DNA virus replicating in the cytoplasm, does not interfere with miRNA pathways, allowing the development of effective vaccines based on the introduction of microRNA target sites as a strategy to attenuate the virus. This finding was unexpected as ASFV is a large DNA virus replicating in the cytoplasm and it has been described that large DNA viruses replicating in the cytoplasm (i.e. poxvirus) interfere with microRNA (miRNA) pathways, rendering these pathways inefficient (see Backes et al, Cell Host Microbe 2012;12(2):200-10, PMID: 22901540).

The authors have found that the insertion of at least one microRNA (miRNA) target site or sequence into the ASFV genome within a gene that is essential for viral replication, renders the resulting ASFV unable of replicating in viral target cells in which the miRNA is expressed. The recombinant ASFV is, thus, restricted on its ability to replicate and disseminate among animals having viral target cells in which the miRNA is expressed, rendering the recombinant ASFV according to the invention a suitable approach as an effective vaccine composition. In addition, while the recombinant ASFV is unable of replicating in viral target cells expressing the miRNA specific for the miRNA target sequence, the recombinant ASFV is able of replicating in suitable cells types which do not express the specific miRNA, allowing the production of viral particles of the recombinant, (in viral target cells) replication-impaired ASFV.

Thus, in a first aspect the invention relates to a recombinant African swine fever virus (ASFV), characterized in that
(i) the genome of the recombinant ASFV comprises at least one miRNA target sequence,
(ii) the miRNA target sequence is found within a gene that is essential for viral replication and
(iii) the miRNA target sequence is specific for a miRNA which is expressed in a viral target cell.

African swine fever virus (ASFV) refers to the virus identified by Taxonomy ID: 10497 including strains, genetic variants, subtypes, isolates, clones or cultures thereof.

The term "variant", "strain" or "isolate" refers to any genetic variant or subtype of the African swine fever virus (ASFV). Non-limiting examples of ASFV isolates, strains or variants are Arm/07/CBM/c2 (GenBank accession number: LR812933.1), Arm/07/CBM/c4 (GenBank accession number: LR881473.1), NHV (KM262845.1), OURT88/3 (NC_044957.1), Benin 97/1, Chiredzi/83/1/CH1, crocodile/96/1/CR1, crocodile/96/3/CR3, E-70, E-70/MS16, E-70/MS44, E-75, E75, 2007/1, Haiti 811, LIS57, Lisbon 60/lis60, Malawi LIL 20/1, OURT 88/3, Georgia 2007/1 (FR682468.2), CzechRepublic 2017/1 (LR722600.1), Moldova 2017/1 (LR722600.1), Belgium 2018/1 (LR536725.1), Germany 2020/1 (LR899193.1), HU_2018 (MN715134.1), China/2018/AnhuiXCGQ (MK128995.1), SY18 (MH766894.2), ASFV/pig/China/CAS19-01/2019 (MN172368.1), Hanoi 2019 (MT166693.1), Wuham 2019-2 (MN393477.1), Wuham 2019-1 (MN393476.1), ASFV/Timor-Leste/2019/1 (MW396979.1), Estonia 2014 (LS478113.1), MAL/19/Karonga (MW856068.1), Tanzania/Rukwa/2017/1 (LR813622.1), ASFV/Kabardino-Balkaria 19/WB-964 (MT459800.1), GZ201801 (MT496893.1), ASFV_NgheAn_2019 (MT180393.1), ASFV/POL/2015/Podlaskie (MH681419.1), 47/Ss/2008 (NC_044955.1), ASFV/LT14/1490 (MK628478.1), pig/Kenya/KEN-50/1950, Pretoriuskop/96/5/pr5, tick/South Africa/Pretoriuskop Pr4/1996, warthog/Namibia/Wart80/1980, Wildebeeslaagte/96/1/m1 and genotypes thereof (https://www.ncbi.nlm.nih.gov/Taxonomy/Browser/wwwtax.cgi?id=10497), and any additional isolated ASFV full genome.

Without limitation, the term ASFV also includes strains, isolates and variants based on sequence variation in the C-terminal region of the B646L gene encoding the major capsid protein p72.

Suitable ASFV strains to be used as "recombinant ASFV" according to the present invention include any variant of ASFV, the genome of which hybridizes under stringent conditions with the genome of the any of the ASFV strains mentioned above. In general, a low stringency hybridization reaction is carried out at about 40 degrees centigrade in 10xSSC or a solution of equivalent ionic strength/temperature. A moderate stringency hybridization is typically performed at about 50 degrees centigrade in 6x SSC, and a high stringency hybridization reaction is generally performed at about 60 degrees centigrade in 1x SSC.

In another embodiment, suitable ASFV strains to be used as "recombinant ASFV" according to the present invention include any ASFV strain which shows a high average nucleotide identity (ANI) with any of the strains mentioned above, The ANI measure the degree of relatedness between the genome of two organisms based on the conservation of the core genome (Konstantinidis K and Tiedje JM, 2005, Proc. Natl. Acad. Sci. USA 102: 2567-2592). In some embodiments, the ANI between the "recombinant ASFV" and any of the ASFV strains mentioned above is of about 95%, about, 96%, about 97%, of about 98%, of about 99%, of about 99,1%, of about 99,5%, of about 99,6%, of about 99,7%, of about 99,8%, of about 99,9%, of about 99,99%, of about 99,999%, of about 99,9999%, of about 99,99999%, of about 99,999999% or more but less than 100%.

In another embodiment, the degree of relatedness between the "recombinant ASFV" and the ASFV strain from which it derives is determined as the Tetranucleotide Signature Frequency Correlation Coefficient, which is based on oligonucleotide frequencies (Bohlin J. et al. 2008, BMC Genomics, 9:104). In some embodiments, the Tetranucleotide Signature Frequency Correlation coefficient between the "recombinant ASFV" and the ASFV strain from which it derives is of about 0,99, 0,999, 0,9999, 0,99999, 0,999999, 0,999999 or more but less than 1.

In another embodiment, suitable ASFV strains to be used as "recombinant ASFV" according to the present invention show a degree of relatedness with any of the strains defined above which is high when determined by Pulsed-field gel electrophoresis (PFGE) using one or more restriction endonucleases. The degree of similarity obtained by PFGE can be measured by the Dice similarity coefficient. In some embodiments, the Dice similarity coefficient between the "recombinant ASFV" and any of the strains defined above is of about 95%, about, 96%, about 97%, of about 98%, of about 99%, of about 99,1%, of about 99,5%, of about 99,6%, of about 99,7%, of about 99,8%, of about 99,9%, of about 99,99%, of about 99,999%, of about 99,9999%, of about 99,99999%, of about 99,999999% or more but less than 100%.

In another embodiment, suitable ASFV strains to be used as "recombinant ASFV" according to the present invention show a degree of relatedness with any of the strains defined above which is high when determined using a Pearson correlation coefficient obtained by comparing the genetic profiles of both strains obtained by repetitive extragenic palindromic element-based PCR (REP-PCR) (see e.g. Chou and Wang, Int J Food Microbiol. 2006, 110:135-48). In some embodiments, high Pearson correlation coefficient obtained by comparing the REP-PCR profiles of the "recombinant ASFV" and any of the ASFV strains mentioned above is of about 0,99, 0,999, 0,9999, 0,99999, 0,999999, 0,999999 or more but less than 1.

In another embodiment, suitable ASFV strains to be used as "recombinant ASFV" according to the present invention show a short linkage distance with respect to any of the ASFV strains defined above, wherein the linkage distance is obtained by comparing the genetic profiles of both the recombinant and any of the ASFV strains defined above by Multilocus sequence typing (MLST) (see e.g. Maiden, M. C., 1998, Proc. Natl. Acad. Sci. USA 95:3140-3145). In some embodiments, the linkage distance obtained by MLST between the "recombinant ASFV" and any of the ASFV strain defined above is of about 0,99, 0,999, 0,9999, 0,99999, 0,999999, 0,999999 or more but less than 1.

As used herein, the term "viral target cell" may be understood as a cell which can be infected by an ASFV strain *in vivo* and which may allow for a full replication cycle of ASFV to be completed. In some embodiments, the viral target cell is characterized in that it expresses the miRNA which is specific for the at least one miRNA target sequence inserted in the viral genome

The term "genome" refers to the complete genetic material of an organism, in particular a virus, more in particular ASFV. The term "recombinant ASFV" refers to ASFV in which the genome is modified. A "recombinant ASFV", as used herein, refers to any ASFV strain, isolate or variant in which the genome is modified by addition, substitution or modification of one or more nucleotide residues.

As used herein, the term "viral target cell", refers to any cell type that can be infected by the recombinant ASFV according to the invention.

The viral target cell can be a "permissive viral target cell" in which, after the cell has been infected by the recombinant ASFV, the viral replication cycle may progress into a complete cycle resulting in the expression of early and late proteins, formation of new infectious viral particles and their release to the extracellular space. A list of ASFV genes (ORFs) and proteins expressed upon infection by ASFV in "permissive viral target cells" as used herein can be found in Yañez et al, 1995 Virology, 208(1):249-78 and Alejo et al, 2018, J Virol, 92(23):e01293-18, which, without limitation, can be used to identify suitable "permissive viral target cells" in the context of the present invention. ASFV late proteins include, for example, p72 (B646L Gene ID: 41901945), p12 (A137R Gene ID: 41901905), p17 (D117L Gene ID: 41901970) and CD2v (EP402R Gene ID: 41901921), which, without limitation, can be used to identify suitable "permissive viral target cells" in the context of the present invention.

In other embodiments, the "virus target cell" is a "restrictive viral target cell" which is a cell in which the recombinant ASFV according to the invention cannot progress into a complete cycle, resulting in an arrest of the viral cycle wherein no viral proteins are expressed or only early viral proteins are expressed but without resulting in the formation of newly assembled viral particles. It will be understood that the "restrictive viral target cell" is characterized in that it expresses the miRNA specific for the at least one miRNA target sequence inserted in the viral genome of the recombinant ASFV of the invention. In a preferred embodiment, the restrictive viral target cell is a porcine alveolar macrophage.

As used herein, the terms "recombinant African swine fever virus" and "recombinant ASFV" are used interchangeably and they refer to a specifically developed variant of any African swine fever virus strain in which the structural and functional features of the ASFV strain from which the "recombinant ASFV" derives are substantially preserved, so that it can replicate permissive viral target cells in cells other than viral target cells. The term "recombinant ASFV" also refers to a specifically developed variant of any African swine fever virus strain which is unable to complete a full replication cycle in restrictive viral target cells.

Methods for detecting or identifying the structural features and activity of the "recombinant ASFV" of the invention based on the presence or expression of ASFV nucleic acids and/or ASFV proteins in a given sample are known in the art and include, without limitation, PCR amplification, RT-PCR, Sanger sequencing and next generation sequencing (NGS), Western blotting etc. These techniques and other known in the art can be used to differentiate or compare the recombinant ASFV with respect to the ASFV strain from which the recombinant ASFV derives.

In some embodiments, the recombinant ASFV according to the present invention shows a reduction in its ability to replicate in restrictive viral target cells with respect to the permissive target cells ASFV by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99% or more.

In some embodiments, the recombinant ASFV according to the present invention shows a reduction in its ability to replicate in restrictive target cells with respect to the non-recombinant ASFV by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99% or more.

In some embodiments, the recombinant ASFV according to the present invention is characterized in that, when used to infect restrictive viral target cells, results in the expression of one or more of late ASFV proteins (including p72, p17 and/or p12 proteins) which is reduced by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99% or more with respect to the expression said one or more late ASFV proteins when the recombinant ASFV is used to infect permissive viral target cells.

In some embodiments, the recombinant ASFV according to the present invention is characterized in that, when used to infect restrictive viral target cells, results in the expression of one or more of the late ASFV proteins (including p72, p17 and/or p12 proteins) which is reduced by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99% or more with respect to the expression of said late ASFV proteins when the cells are infected with the non-recombinant ASFV.

In some embodiments, the recombinant ASFV according to the present invention is characterized in that, when used to infect restrictive viral target cells, results in a reduction in the ASFV DNA content in the cells which of at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99% or more with respect to the DNA content when the recombinant ASFV is used to infect permissive viral target cells.

In some embodiments, the recombinant ASFV according to the present invention is characterized in that, when used to infect restrictive viral target cells, results in a reduction in the ASFV DNA content in the cells which is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99% or more with respect to the DNA content present in cells infected with the non-recombinant ASFV.

According to the invention, the genome of the "recombinant ASFV" comprises a least one "miRNA target site".

The term "miRNA target", "miRNA target sequence" or "miRNA target site" is understood as a region of the genome of the recombinant ASFV which can be transcribed resulting in an mRNA in which the sequence derived from the miRNA target site is specifically recognized and bound by a given miRNA. A given miRNA is complementary to the target mRNA sequence and hybridizes to the target mRNA sequence inhibiting its translation. According to the present invention, the "miRNA target site" comprises a sequence of nucleotides specifically designed to correspond to miRNA which is expressed in the restrictive viral target cell.

In some embodiments, the miRNA target sequence comprises at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides, at least 22 nucleotides, at least 23 nucleotides, at least 24 nucleotides, at least 25 nucleotides, at least 26 nucleotides, at least 27 nucleotides, at least 28 nucleotides, at least 29 nucleotides, at least 30 nucleotides, at least 35 nucleotides, at least 40 nucleotides, at least 45 nucleotides, at least 50 nucleotides, at least 60 nucleotides, at least 70 nucleotides, at least 80 nucleotides, at least 90 nucleotides, at least 100 nucleotides, or at least 200 nucleotides.

In a preferred embodiment, the miRNA target sequence is specific for miRNA-142-3p. In a still more preferred embodiment, the miRNA target sequence comprises, essentially comprises or consist of SEQ ID NO:1.

The present invention contemplates the use of any miRNA expressed in porcine alveolar macrophages (PAMs) for the design of specific miRNA target sequences. A list of suitable miRNAs which may be used according to the present invention can be found in Liu et al, 2016 PLoS One, 11(3):e0150971 and Li et al, 2015, Virol J, 12:128.

The term "viral replication" refers to the process by which viral particles, in particular, recombinant ASFV particles, are produced during infection in viral target cells.

The term "gene essential for viral replication" is understood as any gene of ASFV which is indispensable to allow a full cycle of viral replication to be completed in a permissive target cell. These genes are characterized in that, if they are silenced preferably by miRNA interfering, results in impaired viral DNA replication and/or protein expression, leading to inhibition of viral replication. A list of non-limiting examples of essential genes within the meaning of the invention is shown in Table 1.

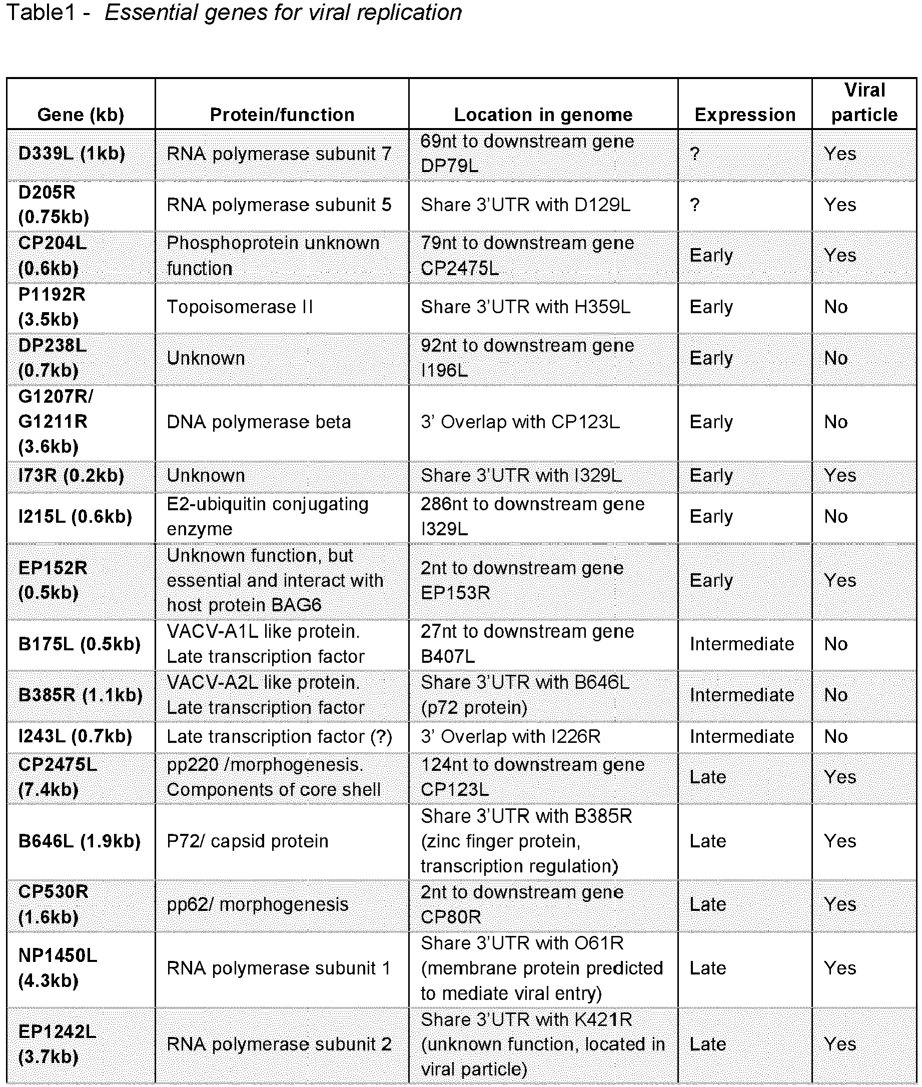

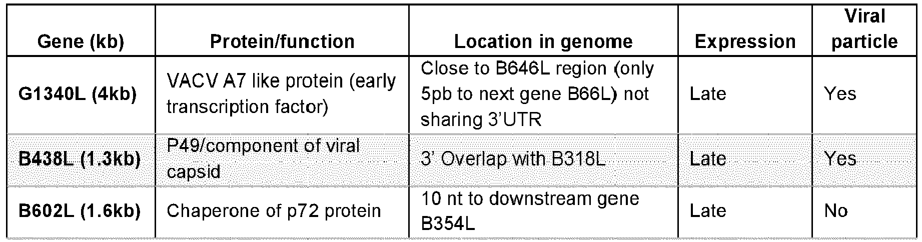

The terms "microRNA" or "miRNA" or "miRNAs" or "miR", used interchangeably herein, are endogenous RNAs, some of which are known to regulate the expression of protein-coding genes at the post-transcriptional level. As used herein, the term "miRNA" refers to any type of micro-interfering RNA, including but not limited to, endogenous microRNA and artificial microRNA. Typically, endogenous miRNAs are small RNAs encoded in the genome which are capable of modulating the productive utilization of mRNA. The term "miRNA", as used herein, may refer to "cell-specific miRNA". In some embodiment, the term "miRNA", as used herein, may refer to "porcine alveolar macrophage-specific miRNA".

In a preferred embodiment, the miRNA target site is inserted within the gene that is essential for viral replication in the region which encodes the 3'-untranslated region (3'-UTR) of the corresponding mRNA.

In another preferred embodiment, multiple copies of the miRNA target sequence are inserted in tandem.

In some embodiments, at least 2 copies, at least 3 copies, at least 4 copies, at least 5 copies, at least 6 copies, at least 7 copies, at least 8 copies, at least 9 copies, or at least 10 copies of the miRNA target sequence are inserted in tandem.

In some embodiments, multiple copies of the same miRNA target sequence are inserted in tandem. In other embodiments, multiple copies of different miRNA target sequences are inserted in tandem.

The term "in tandem" as used herein refers to the adjacent location of copies of the miRNA target sequence within the genome of the recombinant ASFV.

In some embodiments, multiple miRNA target sites can be located in different essential genes. In some embodiments, recombinant ASFV genome contains at least 2, at least 3, at least 4, at least 5, at least 6, at least 7, at least 8, at least 9, at least 10 or more miRNA target site, each of them being located within a different essential gene. In some embodiments, multiple miRNA target sites can be located in different essential genes, each of the multiple miRNA target sites located individually in each of the multiple essential genes. In some embodiments, multiple miRNA target sites can be located in different essential genes, each of the multiple miRNA target sites located in tandem in each of the multiple essential genes.

In some embodiments, the copies of the miRNA target sequence located in tandem in the genome of the recombinant ASFV are separated to each other by at least 1 nucleotide, at least 2 nucleotides, at least 5 nucleotides, at least 10 nucleotides, at least 20 nucleotides, at least 30 nucleotides, at least 40 nucleotides, at least 50 nucleotides, at least 100 nucleotides, at least 200 nucleotides.

In another preferred embodiment, the gene that is essential for viral replication is the G1211R gene (NCBI: F8213_gp093 G1211R [African swine fever virus] Gene ID: 41901953, updated on 17-Apr-2021). G1211R gene is located in the genome of African swine fever virus strain Arm/07/CBM/c2 genome assembly at 3' overlap with CP123L and encodes the ASVF DNA polymerase beta which is not part of the viral particle.

In another preferred embodiment, the miRNA which is expressed in the restrictive viral target cell, preferably in PAM, is the miRNA-142-3p.

The term "miR-142-3p" or "miRNA-142-3p" or "ssc-miR-142-3p" or "porcine miR-142-3p" as used herein, relates to the miRNA as defined in the mIRBase database of the University of Manchester (http://www.mirbase.org/index.shtml) (release as of September 2021) under accession number MIMAT0020362, as well as to homologs and orthologues thereof).

In another preferred embodiment, the miRNA target sequence is defined in SEQ ID NO:1. It will be understood that the target sequence of the miRNA is defined based on the DNA sequence of the ASFV genome, but that the miRNA acts on the mRNA encoded by the gene which contains the target sequence. Accordingly, the "target sequence" can also be defined as the sequence of SEQ ID NO:2, which is the sequence found in the 3'-UTR of the mRNA which is being silenced. Moreover, the target sequence can be also defined as the sequence which is specifically recognized by the sequence region in the miRNA which hybridizes with the target sequence. Accordingly, in another embodiment, the target sequence is any sequence that can be specifically recognized by the sequence of SEQ ID NO:3, which is present in the mature miRNA and which leads to silencing of the mRNA containing said sequence.

The authors of the present invention contemplate the design of miRNA target sites corresponding to different miRNAs expressed in porcine alveolar macrophages as a suitable approach for providing a recombinant ASFV within the meaning of the invention. Suitable miRNAs expressed in porcine alveolar macrophages can be identified by conventional methods.

As used herein, a "cell expressing a miRNA" can be identified by detecting the presence in the cell of the miRNA itself, of the corresponding pre-miRNA or by means of functional assays detecting the silencing of a reporter gene that contains the target sequence of the miRNA in its 3'-UTR. A suitable assay for detecting the expression of a miRNA in a cell of interest include, without limitation, assays in which a reporter mRNA is expressed in the cell of interest wherein the reporter mRNA contains a target sequence for the miRNA, whereby the loss of expression of the reporter gene is indicative that the cell of interest expresses the miRNA.

In one embodiment, the miRNA target sequence that comprises the sequence SEQ ID NO:1 is inserted within the G1211R gene in the region which encodes the 3'-UTR of the corresponding mRNA.

In another embodiment, the genome of the recombinant ASFV contains several copies in tandem of the miRNA target sequence. In some embodiment, the recombinant ASFV contains at least two, at least three, at least four, at least five, at least six, at least eight, at least ten, at least 20, at least 30, at least 40 or more copies in tandem of the miRNA target sequence. In one embodiment, the genome of the recombinant ASFV contains four copies in tandem of the miRNA-142-3p target sequence at the 3'-UTR of the G1211R gene. In a further preferred embodiment, the miRNA target sequence is defined in SEQ ID NO:4.

In a preferred embodiment, the recombinant ASFV is derived from the hemadsorbing ASFV isolate Arm/07 genotype II (Pérez-Núñez et al., Vaccines 2020, 8, 625).

In another preferred embodiment, the recombinant ASFV is derived from the non-hemadsorbing ASFV isolate NH/P68 genotype I (Gallardo et al. 2018, Vaccine, 36(19):2694-2704, PMID: 29609966).

In a further preferred embodiment, the recombinant ASFV genome is derived from the ASFV Arm/07/CBM/c2 strain, having the genome as defined in the GenBank accession number LR812933.1 (Pérez-Núñez et al., 2020 Vaccines, 8, 625, Assembled genome Arm/07/CBM/c2 together with the annotation deposited in the European Nucleotide Archive (ENA) under the study accession number PRJEB38146. Raw reads from clones 1 and 3 deposited under the study accession PRJEB40011).

In one embodiment, the recombinant ASFV further comprises a reporter gene. In another embodiment, the reporter gene is under the control of a promoter of a late ASFV gene. In another embodiment, the reporter gene is operably linked to the p72 promoter. In another embodiment, the reporter gene is operably linked to the EP402R promoter of ASFV.

Useful reporter genes in the context of the present invention include GFP, Dsred, lacZ, luciferase, thymidine kinase and the like. Useful marker genes in the context of this invention include, for example, the neomycin resistance gene, conferring resistance to the aminoglycoside G418; the hygromycin phosphotransferase gene, conferring resistance to hygromycin; the ODC gene, conferring resistance to the inhibitor of the ornithine decarboxylase (2-(difluoromethyl)-DL-ornithine (DFMO); the dihydrofolatereductase gene, conferring resistance to methotrexate; the puromycin-N-acetyl transferase gene, conferring resistance to puromycin; the ble gene, conferring resistance to zeocin; the adenosine deaminase gene, conferring resistance to 9-beta-D-xylofuranose adenine; the cytosine deaminase gene, allowing the cells to grow in the presence of N-(phosphonacetyl)-L-aspartate; thymidine kinase, allowing the cells to grow in the presence of aminopterin; the xanthine-guanine phosphoribosyltransferase gene, allowing the cells to grow in the presence of xanthine and the absence of guanine; the trpB gene of E. coli, allowing the cells to grow in the presence of indol instead of tryptophan; the hisD gene of E.coli, allowing the cells to use histidinol instead of histidine. The selection gene is incorporated into a plasmid that can additionally include a promoter suitable for the expression of said gene in eukaryotic cells (for example, the CMV or SV40 promoters), an optimized translation initiation site (for example, a site following the so-called Kozak's rules or an IRES), a polyadenylation site such as, for example, the SV40 polyadenylation or phosphoglycerate kinase site, introns such as, for example, the beta-globulin gene intron. Alternatively, it is possible to use a combination of both the reporter gene and the marker gene simultaneously in the same vector.

### Polynucleotide according to the invention, and vectors and host cells containing said polynucleotide

In another aspect, the invention relates to a polynucleotide comprising a first, second and third regions, wherein the first region comprises the region of an ASFV gene which encodes the 3'-UTR of the mRNA encoded by said gene, which region is modified by replacing at least part of said region by at least one miRNA target site, wherein the first region is flanked by the second and third regions, and wherein said second and third regions are the ASFV genomic regions which naturally flank the first genomic region in the ASFV genome.

As used herein, the term "polynucleotide" refers to a polymer composed of a multiplicity of nucleotide units (deoxyribonucleotides or ribonucleotides, or related structural variants or synthetic analogues thereof) linked via phosphodiester bonds (or related structural variants on synthetic analogues thereof). The term polynucleotide includes double or single stranded genomic and cDNA, RNA, any synthetic and genetically manipulated polynucleotide, and both sense and anti-sense polynucleotide (although only sense stands are being disclosed in the present invention). This includes single- and double-stranded molecules, i.e., DNA-DNA, DNA-RNA and RNA-RNA hybrids.

Recombinant techniques may be used to generate the polynucleotide of the present invention. To produce a polynucleotide of the present invention using recombinant technology, a polynucleotide comprising a first region comprising the region of an ASFV gene which encodes the 3'-UTR of the mRNA encoded by said gene, which region is modified by replacing at least part of said region by at least one miRNA target site according to the present invention, wherein the first region is flanked by second and third regions, can be generated using conventional techniques. It will be understood that the polynucleotide according to the invention may contain in the first region only that region of the gene that encodes the 3'-UTR. In other embodiments, the first region of the polynucleotide of the invention contains the complete ASFV gene. In some embodiments, the first region of the polynucleotide of the invention contains a region of the ASFV genome which comprises the gene in which the region encoding the 3'-UTR is modified by the insertion of at least one miRNA target site as well as the partial or complete sequences of neighboring genes at 5' or 3' position in the ASFV genome with respect to the gene in which the region encoding the 3'-UTR is modified by the insertion of at least one miRNA target site.

As used herein, the term "the first region comprises the region of an ASFV gene which encodes the 3'-UTR of the mRNA encoded by said gene" refers to the sequence of nucleotides of an ASFV gene that contains the sequence which is needed for the transcription into a complete or partial 3'-UTR of the ASFV gene.

As used herein, the term "region is modified by replacing at least part of said region by at least one miRNA target site" refers to the inclusion or addition of one or more nucleotide target sequences within or at the vicinity of the region or part of the ASFV gene that is necessary and sufficient for transcription of a mRNA molecule comprising a complete or partial 3'-UTR of the ASFV gene, which one or more nucleotide target sequences, upon transcription into a mRNA molecule comprising the so modified 3'-UTR, can be recognized and bound by a given miRNA.

As used herein, the term "inclusion of one or more nucleotide target sequences within or at the vicinity of the region or part of the ASFV gene encoding a 3'-UTR of the ASFV gene" refers to the insertion of one or more miRNA nucleotide target sequences at one or the opposite end of the region or part of the ASFV gene encoding the 3'-UTR, and/or to the insertion of nucleotide target sequences amid the sequence of nucleotides forming the region or part of the ASFV gene encoding the 3'-UTR of the ASFV gene. It will be understood that the miRNA target site may be inserted between two specific nucleotides of the region of the ASFV encoding the 3'-UTR but maintaining all nucleotides of the region encoding the 3'-UTR. In other embodiments, the miRNA target site may be inserted replacing one or more nucleotides of the region of the ASFV encoding the 3'-UTR. In other embodiments, the insertion of the miRNA target site or of the region containing several copies in tandem of the miRNA target site results in the incorporation of one or more nucleotides at the junctions between the region encoding the 3'-UTR region and the miRNA target sites. The insertion may consist of 1, 2, 3, 4, 5, 6, 7, 8, 9 10, 20, 30 or more nucleotides and can be present at the 5' junction, at the 3' junction or at both the 5' and 3' junction regions.

As used herein "at the vicinity of a region" is understood as a location in a polynucleotide sequence which is at a distance of at least 1 nucleotide, at least 2 nucleotides, at least 3 nucleotides, at least 4 nucleotides, at least 5 nucleotides, at least 10 nucleotides, at least 20 nucleotides, at least 30 nucleotides, at least 40 nucleotides, at least 50 nucleotides, at least 100 nucleotides, to said region.

As used herein, the term "first region is flanked by the second and third regions" refers to the relative location of the second and third regions with respect to the first region. In some embodiments, the second region is located at one given end of the first region corresponding to the 5' end of the first region, and the third region is located at the opposite end of the first region corresponding to the 3'end of the first region. In some embodiments, the second region is located at one given end of the first region corresponding to the 3' end of the first region, and the third region is located at the opposite end of the first region corresponding to the 5'end of the first region. In some embodiments, the second and third regions are located respectively adjacent to the first region. In some embodiments, the second and third regions are located respectively not adjacent to the first region. In some embodiment, second and third regions which are not adjacent to the first region are separated respectively to the first region by at least 1 nucleotide, at least 2 nucleotides, at least 3 nucleotides, at least 4 nucleotides, at least 5 nucleotides, at least 10 nucleotides, at least 20 nucleotides, at least 30 nucleotides, at least 40 nucleotides, at least 50 nucleotides, at least 100 nucleotides, at least 200 nucleotides, at least 500 nucleotides, at least 1000 nucleotides.

The term "said second and third regions are the ASFV genomic regions which naturally flank the first genomic region in the ASFV genome" as used herein refer to the corresponding nucleotide sequences and their relative location as found in the genome of an unmodified ASFV strain.

In some embodiments, the miRNA target site comprises at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides, at least 22 nucleotides, at least 23 nucleotides, at least 24 nucleotides, at least 25 nucleotides, at least 26 nucleotides, at least 27 nucleotides, at least 28 nucleotides, at least 29 nucleotides, at least 30 nucleotides, at least 35 nucleotides, at least 40 nucleotides, at least 45 nucleotides, at least 50 nucleotides, at least 60 nucleotides, at least 70 nucleotides, at least 80 nucleotides, at least 90 nucleotides, at least 100 nucleotides, or at least 200 nucleotides.

In a preferred embodiment, the miRNA target site comprised in the polynucleotide according to the invention is specific for miR-142-3p. In a still more preferred embodiment, the miRNA target site comprised in the polynucleotide according to the invention comprises, essentially comprises or consist of SEQ ID NO:1.

In some embodiments, the polynucleotide according to the invention comprises multiple copies of the miRNA target site which are arranged in tandem.

In some embodiments, at least 2 copies, at least 3 copies, at least 4 copies, at least 5 copies, at least 6 copies, at least 7 copies, at least 8 copies, at least 9 copies, or at least 10 copies of the miRNA target sequence are inserted in tandem.

In some embodiments, multiple copies of the same miRNA target site are inserted in tandem. In other embodiments, multiple copies of different miRNA target site are inserted in tandem.

The term "in tandem" as used herein refers to the adjacent location of copies of the miRNA target site inserted within or at the vicinity of the region or part of the an ASFV gene encoding the 3'-UTR of the gene.

In some embodiments, multiple miRNA target sequences can be inserted within or at the vicinity of the respective region or part of different ASFV genes encoding the 3'-UTR of the respective ASFV gene.

In some embodiments, multiple miRNA target sequences can be inserted within or at the vicinity of the respective region or part of different ASFV genes encoding the 3'-UTR of the respective ASFV gene, each of the multiple miRNA target sequences inserted individually within or at the vicinity of the respective region or part of the different ASFV genes encoding the 3'-UTR of the respective ASFV gene.

In some embodiments, multiple miRNA target sequences can be inserted within or at the vicinity of the respective region or part of different ASFV gene encoding the 3'-UTR of the respective ASFV gene, each of the multiple miRNA target sequences inserted in tandem within or at the vicinity of the respective region or part of the different ASFV genes encoding the 3'-UTR of the respective ASFV gene.

In some embodiments, the copies of the miRNA target site inserted in tandem within or at the vicinity of the 3'-UTR of an ASFV gene are separated to each other by at least 1 nucleotide, at least 2 nucleotides, at least 5 nucleotides, at least 10 nucleotides, at least 20 nucleotides, at least 30 nucleotides, at least 40 nucleotides, at least 50 nucleotides, at least 100 nucleotides, at least 200 nucleotides.

In a preferred embodiment, the polynucleotide according to the invention is a deoxyribonucleic acid (DNA) polynucleotide.

In a preferred embodiment, the region or part of the an ASFV gene encoding the 3'-UTR of the gene comprised in the polynucleotide according to the invention belongs to an ASFV gene which is an essential gene for viral replication.

In a preferred embodiment, the region or part of the an ASFV gene encoding the 3'-UTR of the gene comprised in the polynucleotide according to the invention belongs to the ASFV G1211R gene. In another embodiment, the region or part of the an ASFV gene encoding the 3'-UTR of the gene comprised in the polynucleotide according to the invention belongs to the ASFV B646L gene. In another embodiment, the region or part of the an ASFV gene encoding the 3'-UTR of the gene comprised in the polynucleotide according to the invention belongs to the ASFV 602L gene.

In some embodiments, the polynucleotide according to the invention comprises four copies in tandem of the miRNA-142 target site inserted within or at the vicinity of the region or part of the ASFV G1211R gene encoding the 3'-UTR of the gene.

In some embodiments, the polynucleotide according to the invention comprises four copies in tandem of the miRNA-142 target site inserted within or at the vicinity of the region or part of the ASFV B646L gene encoding the 3'-UTR of the gene.

In some embodiments, the polynucleotide according to the invention comprises four copies in tandem of the miRNA-142 target site inserted within or at the vicinity of the region or part of the ASFV 602L gene encoding the 3'-UTR of the gene.

In a preferred embodiment, the polynucleotide according to the invention comprises the complete sequence of the an ASFV gene from which the region or part encoding the modified 3'-UTR of the gene derives, which ASFV gene is essential for viral replication.

In another preferred embodiment, the polynucleotide of the invention comprises the complete sequence of the an ASFV gene from which the region or part of the ASFV gene encoding the 3'-UTR of the gene derives, which ASFV gene is selected from the group comprised by the ASFV genes G1211R, B646L or 602L.

The authors of the present invention contemplate the design of miRNA target sites corresponding to different miRNAs expressed in porcine alveolar macrophages as a suitable approach for providing a polynucleotide within the meaning of the invention. Suitable miRNAs expressed in porcine alveolar macrophages can be identified by conventional methods.

In a further aspect, the invention relates to a vector comprising the polynucleotide comprising a first, second and third regions, wherein the first region comprises the region of an ASFV gene which encodes the 3'-UTR of the mRNA encoded by said gene, which region is modified by replacing at least part of said region by at least one miRNA target site, wherein the first region is flanked by the second and third regions and wherein said second and third regions are the ASFV genomic regions which naturally flank the first genomic region in the ASFV genome. The vector comprising the polynucleotide according to the invention finds application in the production of the recombinant ASFV of the invention. In some embodiments, the polynucleotide sequences comprised in the vector of the invention are required for the production of the recombinant ASFV of the invention.

Vectors suitable for the cloning of said polynucleotide are vectors derived from expression vectors in prokaryotes such as pUC18, pUC19, Bluescript and the derivatives thereof, mpl8, mpl9, pBR322, pMB9, ColEI, pCRI, RP4, phages and "shuttle" vectors such as pSA3 and pAT28; expression vectors in yeasts such as vectors of the type of 2 micron plasmids, integration plasmids, YEP vectors, centromere plasmids and the like; expression vectors in insect cells such as vectors of the pAC and pVL; expression vectors in plants such as plBI, pEarleyGate, pAVA, pCAMBIA, pGSA, pGWB, pMDC, pMY, pORE series and the like; and expression vectors in eukaryotic cells, including baculovirus suitable for transfecting insect cells using any commercially available baculovirus system such as pFastBac1. The vectors for eukaryotic cells include preferably viral vectors (adenoviruses, viruses associated to adenoviruses such as retroviruses and, particularly, lentiviruses) as well as non-viral vectors such as pSilencer 4.1-CMV (Ambion), pcDNA3, pcDNA3.1/hyg, pHMCV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and PKSV-10, pBPV-1, pML2d and pTDTI.

In some embodiments, the vector of the invention further comprises a polynucleotide sequence coding for a detectable marker or reporter. Useful reporter genes in the context of the present invention include GFP, Dsred, lacZ, luciferase, thymidine kinase and the like. Useful marker genes in the context of this invention include, for example, the neomycin resistance gene, conferring resistance to the aminoglycoside G418; the hygromycin phosphotransferase gene, conferring resistance to hygromycin; the ODC gene, conferring resistance to the inhibitor of the ornithine decarboxylase (2-(difluoromethyl)-DL-ornithine (DFMO); the dihydrofolatereductase gene, conferring resistance to methotrexate; the puromycin-N-acetyl transferase gene, conferring resistance to puromycin; the ble gene, conferring resistance to zeocin; the adenosine deaminase gene, conferring resistance to 9-beta-D-xylofuranose adenine; the cytosine deaminase gene, allowing the cells to grow in the presence of N-(phosphonacetyl)-L-aspartate; thymidine kinase, allowing the cells to grow in the presence of aminopterin; the xanthine-guanine phosphoribosyltransferase gene, allowing the cells to grow in the presence of xanthine and the absence of guanine; the trpB gene of *E. coli,* allowing the cells to grow in the presence of indol instead of tryptophan; the hisD gene of E.coli, allowing the cells to use histidinol instead of histidine. The selection gene is incorporated into a plasmid that can additionally include a promoter suitable for the expression of said gene in eukaryotic cells (for example, the CMV or SV40 promoters), an optimized translation initiation site (for example, a site following the so-called Kozak's rules or an IRES), a polyadenylation site such as, for example, the SV40 polyadenylation or phosphoglycerate kinase site, introns such as, for example, the beta-globulin gene intron. Alternatively, it is possible to use a combination of both the reporter gene and the marker gene simultaneously in the same vector.

In some embodiments, the vector comprising the polynucleotide sequence coding for a detectable marker or reporter finds application in the production of the recombinant ASFV of the invention. In some embodiments, the produced recombinant ASFV of the invention comprises the polynucleotide sequence coding for a detectable marker or reporter derived from the vector of the invention. In some embodiments, the presence in the recombinant ASFV of the invention of the polynucleotide sequence coding for a detectable marker or reporter derived from the vector of the invention allows, upon expression of the detectable marker, the isolation or purification of the recombinant ASFV particles, for example and without limitation, by Fluorescence Activated Cell Sorting (FACS).

The polynucleotide of the invention can be introduced into the host cell as naked DNA plasmids, but also using vectors by methods known in the art, including but not limited to transfection, electroporation (e.g. transcutaneous electroporation), microinjection, transduction, cell fusion, DEAE dextran, calcium phosphate precipitation, use of a gene gun, or use of a DNA vector transporter. Other molecules are also useful for facilitating transfection of a nucleic acid, such as cationic oligopeptides, peptides derived from DNA binding proteins, or cationic polymers.

Another well-known method that can be used to introduce polynucleotides into host cells is particle bombardment (aka biolistic transformation). Biolistic transformation is commonly accomplished in one of several ways. One common method involves propelling inert or biologically active particles at cells.

Alternatively, the vector can be introduced in cells by lipofection. The use of cationic lipids can promote encapsulation of negatively charged nucleic acids, and also promote fusion with negatively charged cell membranes. Particularly useful lipid compounds and compositions for transfer of nucleic acids have been described.

In some embodiments, the generation of vectors for the production of the recombinant ASFV of the invention is carried out by means of CRISPR-Cas9 technology.

In one preferred embodiment, the vectors according to the invention comprises a sequence optimized for expression in mammalian cells.

On the other hand, as the skilled person in the art is aware that the choice of the vector will depend on the host cell in which it will subsequently be introduced. By way of example, the vector in which said polynucleotide is introduced can also be a yeast artificial chromosome (YAC), a bacterial artificial chromosome (BAC) or a PI-derived artificial chromosome (PAC). The characteristics of the YAC, BAC and PAC are known by the person skilled in the art. The vector of the invention can be obtained by conventional methods known by persons skilled in the art.

In a further aspect, the invention relates to a host cell comprising the polynucleotide or the vector of the invention.

The term "host cell", as used herein, refers to a cell into which a nucleic acid of the invention, such as a polynucleotide or a vector according to the invention, has been introduced and is capable of producing the recombinant ASFV.

In a preferred embodiment, the "host cell" is a permissive viral target cell. In yet another embodiment, the permissive viral target cell does not express the miRNA corresponding to the miRNA target sequence located in the genome of the recombinant AFSV.

The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. The term includes any cultivatable cell that can be modified by the introduction of heterologous DNA. Preferably, a host cell is one in which the polynucleotide of the invention can be stably expressed, post-translationally modified, localized to the appropriate subcellular compartment, and made to engage the appropriate transcription machinery. The choice of an appropriate host cell will also be influenced by the choice of detection signal. For example, reporter constructs, as described above, can provide a selectable or screenable trait upon activation or inhibition of gene transcription in response to a transcriptional regulatory protein; in order to achieve optimal selection or screening, the host cell phenotype will be considered. A host cell of the present invention includes eukaryotic cells including but not limited to mammalian cells. Mammalian host cell culture systems include established cell lines such as COS cells, L cells, 3T3 cells, Chinese hamster ovary (CHO) cells, embryonic stem cells, with BHK, HeK or HeLa, insect cells, for example but not restricted to, BTI-Tn-5B1-4 (Thermo Fisher Scientific) being preferred. Eukaryotic cells are, preferably, used to for recombinant gene expression by applying the transcription control sequence or the expression vector of the present invention.

The vectors may also comprise a reporter or marker gene as described above which allows identifying those cells that have incorporated the vector after having been put in contact with it.

### Immunogenic composition and vaccine of the invention

The recombinant ASFV of the invention may be used to elicit an immune response against a virulent ASFV or to protect animals against ASFV infection.

In a further aspect, the invention relates to an immunogenic composition or a vaccine composition comprising the recombinant ASFV, the polynucleotide or the vector according to the invention, and a pharmaceutically suitable carrier or excipient.

The recombinant ASFV described herein are useful in the development of immunogenic compositions or vaccines used to elicit a humoral or cellular immune response. Accordingly, the recombinant ASFV and nucleic acids of the invention find application as a prophylactic vaccine, to reduce the replication of ASFV in already infected subjects or limit the infectivity of ASFV in a vaccinated subjects. Such vaccines can include a recombinant ASFV, a nucleic acid molecule encoding said recombinant ASFV or a host cell producing said recombinant ASFV.

According to the present invention, the term "immunogenic composition" refers to a composition able to elicit a specific immune response against ASFV in an animal, in particular, in a domestic pig and/or wild boar.

As used herein, the term "vaccine composition" refers to a composition able to confer protection against ASFV to an animal, in particular, to a domestic pig and/or wild boar.

Suitable assays which may be used to detect if an immune response is generated or if protection is conferred against ASFV according to the present invention can be found in Sanchez et al, (2019, Virus Res, 2019, 265:150-155), and include, for example and without limitation, ELISA assays for detecting specific anti ASFV antibodies in the sera of immunized pigs, ELISAs to detect type I IFN in the sera of immunized animals vs controls, ELISpots detecting IFNgamma in the sera of immunized animals, and the like.

The immunogenic composition or vaccine according to the invention is provided that comprises the recombinant ASFV according to the invention as defined above in a live form, and a pharmaceutically acceptable carrier or diluent. The vaccine according to the invention containing the recombinant ASFV can be prepared and marketed in the form of a suspension or in a lyophilized form and additionally contains a pharmaceutically acceptable carrier or diluent customary used for such compositions. Carriers include stabilizers, preservatives and buffers. Suitable stabilizers are, for example SPGA (sucrose, phosphate, glutamate, and human. albumin), carbohydrates (such as sorbitol, mannitol, starch, sucrose, dextran, glutamate or glucose), proteins (such as dried milk serum, albumin or casein) or degradation products thereof. Suitable buffers are for example alkali metal phosphates. Suitable preservatives are thimerosal, merthiolate and gentamicin. Diluents include water, aqueous buffer (such as buffered saline), alcohols and polyols (such as glycerol).

If desired, the immunogenic composition or vaccine according to the invention may contain an adjuvant. Examples of suitable compounds and compositions with adjuvant activity are well known in the art. Furthermore, nucleic acid sequences encoding polypeptides for pharmaceutical or diagnostic applications, in particular immunomodulators such as lymphokines, interferons or cytokines, may be incorporated into the vaccine.

In some embodiments, the immunogenic composition or vaccine according to the example may contain a suitable adjuvant. Examples of suitable adjuvants include aluminum salts. These adjuvants have been useful for some vaccines including hepatitis B, diphtheria, polio, rabies, and influenza. Other useful adjuvants include Complete Freund's Adjuvant (CFA), Incomplete Freund's Adjuvant (IFA), muramyl dipeptide (MDP), synthetic analogues of MDP, N-acetylmuramyl-L-alanyl-Disoglutamyl-L-alanine-2-[1,2-dipalmitoyl-s-glycero-3-(hydroxyl phosphoryloxy)]ethylamide (MTP-PE) and compositions containing a degradable oil and an emulsifying agent, wherein the oil and emulsifying agent are present in the form of an oil-in-water emulsion having oil droplets all of which are substantially less than one micron in diameter.

The vaccine compositions may be administered in any conventional manner which will introduce the vaccine into the animal, usually by injection. The vaccine of the invention may be administered by any convenient route, such as by intramuscular injection. Other suitable routes of administration include intranasal, oral, subcutaneous, transdermal and vaginal (e.g, during artificial insemination). In one embodiment, oral administration comprises adding the vaccine to animal feed or drinking water. In another embodiment, the vaccine may be added to a bait for a wild animal, for example a bait suitable for wild boar, wild pigs, bushpigs or warthogs. The precise amounts and formulations for use in either prevention or therapy can vary depending on, for example, the circumstances of the inherent purity and activity of the recombinant ASFV, any additional ingredients, carriers or the method of administration.

In some embodiments, the immunogenic compositions and vaccines according to the invention may administered to sows so as transfer maternally-derived antibody (MDA) to the piglets.

### Use of the recombinant ASFV, the polynucleotide, the vector or the vaccine or immunogenic composition in the prevention or treatment of a disease caused by the infection by ASFV

The authors have found that the recombinant ASFV, the polynucleotide, the vector or the vaccine or immunogenic composition of the invention are able to elicit an immune response in a subject, being therefore useful tools for preventing or treating subjects against ASFV infections; thereby, the various clinical symptoms associated to ASFV infection can be prevented, ameliorated or eliminated.

Thus, in another aspect, the invention relates to the recombinant ASFV, the polynucleotide, the vector, or the vaccine or immunogenic composition according to the invention for use in the prevention or treatment of a disease caused by the infection by ASFV.

The term "treatment", as used herein, comprises any type of therapy, which aims at terminating, preventing, ameliorating and/or reducing the susceptibility to a clinical condition as described herein, e.g. ASFV infection and related conditions. Thus, "treatment," "treating," and the like, as used herein, refer to obtaining a desired pharmacologic and/or physiologic effect, covering any treatment of a pathological condition or disorder in a mammal, in particular a domestic pig. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter. "Treatment" can also mean prolonging survival as compared to expected survival if not receiving treatment. Those in need of treatment include those already with the condition or disorder as well as those prone to have the condition or disorder or those in which the condition or disorder is to be prevented.

In another aspect, the invention relates to a method of inducing a protective immune response to an ASFV infection in an animal, said method comprising administering to said animal the recombinant ASFV, the polynucleotide, the vector or the vaccine composition according to invention.

The term "animal" is understood as a mammalian subject for whom protective immune response to an ASFV infection is desired. The term "animal" includes domestic animals, farm animals, zoo animals or pet animals. In a preferred embodiment of the invention, the animal is a mammal. In a more preferred embodiment of the invention, the animal is a domestic pig.

The term "inducing a protective immune response", as used herein, comprises any type of "treatment", which aims at preventing, ameliorating and/or reducing the susceptibility to a clinical condition as described herein, e.g. ASFV infection and related conditions, by eliciting an immune response.

### Method for producing the recombinant ASFV of the invention

The authors have developed methods for producing the recombinant ASFV of the invention using permissive viral target cells. When the recombinant ASFV is produced using a polynucleotide according to the invention, an ASFV strain and means for creating a double strand break in the genome of said ASFV strain, the recombinant ASFV particles are secreted into the cell culture media, from which they can be recovered, isolated and/or purified. Thus, in a further aspect, the invention relates to a method for producing a recombinant African swine fever virus (ASFV), the method comprising:
(i) modifying permissive viral target cells by
   - introducing a polynucleotide comprising a first, second and third regions, wherein the first region comprises the region of an ASFV gene essential for viral replication which encodes the 3'-UTR of the mRNA encoded by said gene, which region is modified by replacing at least part of said region by at least one miRNA target site, wherein the first region is flanked by the second and third regions and wherein said second and third regions are the ASFV genomic regions which naturally flank the first genomic region in the ASFV genome, wherein the polynucleotide further comprises a reporter gene between the second and third region,

   - infecting the cells with an ASFV strain in which the gene that is essential for viral replication does not contain a miRNA target site, and
   - introducing means capable of creating a double strand DNA break in the genome of said ASFV strain within or at the vicinity of the region encoding the 3'UTR of mRNA encoded by the essential gene for viral replication,
(ii) maintaining the cells under conditions adequate for the double-strand DNA break in the ASFV genome to take place and to allow homologous recombination between the ASFV genome containing of the DNA break and the second and third regions of the polynucleotide thereby resulting in the replacement of the region encoding the 3'UTR of mRNA encoded by the ASFV gene essential for viral replication by the first region of the polynucleotide and the reporter gene, and
(iii) recovering the recombinant ASFV from the supernatant and/or from the whole cell extract and selecting the ASFV viruses which contain the reporter gene.

"Permissive viral target cell" according to the invention is any cell type able to produce and secrete ASFV viral particles into the supernatant surrounding the cell, and which do not express the miRNA, the target sequence of which is present in the genome of the recombinant ASFV, for example, but not limited to COS-1, COS-7, WSL-R, WSL-Bu, WSL, PK15/ST.

The term "modifying permissive viral target cells" refers to any manipulation of the permissive viral target cells, including but not restricted to methods of genetic manipulation, which renders the permissive viral target cells capable of effectively producing and/or secreting recombinant ASFV virions.

In a preferred embodiment, the permissive viral target cell is a mammalian cell line. In a further preferred embodiment, the permissive viral target cells are COS cells.

The term "the polynucleotide further comprises a reporter gene between the second and third region" refers to the relative location of a reporter gene with respect to the second and third regions within the polynucleotide according to the invention. In some embodiments, the reporter gene is located between the 3' end of the second region and the 5' end of the third region. In other embodiments, the reporter gene is located between the 5' end of the second region and the 3' end of the third region. In some embodiments, the reporter gene is separated respectively from the second and third regions by at least 1 nucleotide, at least 2 nucleotides, at least 3 nucleotides, at least 4 nucleotides, at least 5 nucleotides, at least 10 nucleotides, at least 20 nucleotides, at least 30 nucleotides, at least 40 nucleotides, at least 50 nucleotides, at least 100 nucleotides, at least 200 nucleotides, at least 500 nucleotides, at least 1000 nucleotides.

The term "means capable of creating a double-strand DNA break in the genome of said ASFV strain" refers to any system comprising t least one polynucleotide, at least one polypeptide and/or a combination thereof which can both specifically target a region of the ASFV genome and create a complete disruption of the double-strand DNA within or at the vicinity of the targeted region.

The term "introducing means capable of creating a double-strand DNA break in the genome of said ASFV strain" refers to any method of providing said means within the intracellular space of the permissive viral target cell, for example but not restricted to methods of transfecting polynucleotides.

In a preferred embodiment, the means capable of creating a double strand break in the genome of said ASFV strain within or at the vicinity of the region encoding the 3'UTR of mRNA encoded by the essential gene for viral replication comprise a CRISPR/Cas system. In a further preferred embodiment, the system may be a CRISPR/Cas 9 system.

According to the invention, upon disruption of the ASFV genome, the presence of second and third regions in the polynucleotide, which correspond to regions naturally occurring in the ASFV genome, allows a homologous recombination process between the ASFV genome and the polynucleotide. The location of second and third regions flanking a first region according to the invention, allows the substitution of the region in the ASFV genome which encodes the 3'-UTR of the mRNA of an ASFV gene essential for viral replication with a modified region which no longer encodes the 3'-UTR of the mRNA but comprises instead at least one miRNA target site and a reporter gene.

The term "maintaining the cells under conditions adequate for the double-strand DNA break in the ASFV genome to take place and to allow homologous recombination" refers to those conditions which allow the complete disruption of the ASFV genome at a specific region of the double-strand DNA and the subsequent homologous recombination process between the disrupted ASFV genome and the polynucleotide of the invention, thereby obtaining a recombinant ASFV having a no longer disrupted genome which comprises polynucleotide sequences comprising the at least one miRNA target site and reporter gene derived from the polynucleotide according to the invention.

"Conditions adequate for the generation of ASFV particles" are those conditions which allow the complete replication of the virus in the cytoplasm of the cells of the expression system, and/or the secretion of recombinant ASFV virions from the cells of the expression system into the supernatant surrounding the cells.

According to the present invention, the recombinant ASFV may be understood as a recombinant protein, wherein the term "recombinant", as used herein, refers to a recombinant ASFV produced using genetic engineering approaches at any stage of the production process, for example by engineering the sequence of the recombinant ASFV itself. The person skilled in the art is familiar with methods for engineering nucleic acids and proteins encoded. In another preferred embodiment, the recombinant ASFV is an isolated recombinant ASFV, wherein the term "isolated" or "recovered" means that the recombinant ASFV has been enriched compared to its state upon production using a biotechnological or synthetic approach and is preferably pure, i.e. at least 60, 70, 80, 90, 95 or 99 percent of the recombinant ASFV in the respective liquid consists of said recombinant ASFV.

"Recovering the recombinant ASFV" refers to any method of protein purification. In some embodiments, the recombinant ASFV of the present invention is provided in the form of a preparation containing isolated recombinant ASFV particles. As used herein, the terms "select", "selected", "selecting", "isolate," "isolated", "isolating", "purify", "purified" or "purifying" as well as "extracted" and "extracting" are used interchangeably and refer to the state of a preparation (e.g., a plurality of known or unknown amount and/or concentration) of recombinant ASFV particles that have undergone one or more processes of purification, e.g., a selection or an enrichment of the desired recombinant ASFV particles.

The term "recovering the recombinant ASFV from the supernatant and/or from the whole cell extract" refers to the collection of recombinant ASFV virions secreted or released from the infected cells into the supernatant of the cell culture and/or to the disruption of infected cells so as to extract or release the recombinant ASFV particles not yet secreted and then to collect the recombinant ASFV virions.

The term "selecting the ASFV viruses which contain the reporter gene" refers to any method of separating the recombinant ASFV, identified on the basis of the expression of the reporter gene, from ASFV viruses which do not contain the reporter gene. It will be understood that the absence of the reporter gene or the product expressed by the reporter gene is indicative that homologous recombination has not taken place and that an ASFV virion which does not comprise the reporter gene is not a recombinant ASFV.

"Recovering the recombinant ASFV" include isolation, purification, and enrichment of the recombinant ASFV particles, which can be done in a general and non-selective manner (typically including serial centrifugation). Alternatively, isolation, purification, and enrichment of recombinant ASFV particles can be done in a more specific and selective manner (e.g., using producer cell-specific surface markers). For example, specific surface markers may be used in immunoprecipitation, FACS sorting, affinity purification, bead-bound ligands for magnetic separation, etc.

In a preferred embodiment, the reporter gene encodes a fluorescent protein. Examples of fluorescent proteins include, but are not restricted to, GFP, Dsred, luciferase.

In another preferred embodiment, the reporter gene is under the control of a constitutive promoter. A constitutive promoter is understood by a skilled person to be a promoter whose expression is constant under the standard culturing conditions, i.e. a promoter which expresses a gene product at a constant expression level.

In some embodiments, size exclusion chromatography can be utilized to isolate or purify the recombinant ASFV particles. Size exclusion chromatography techniques are known in the art. Exemplary, non-limiting techniques are provided herein. In some embodiments, a void volume fraction is isolated and comprises the recombinant ASFV particles. In some embodiments, for example, density gradient centrifugation can be utilized to further isolate the recombinant ASFV particles. Still further, in some embodiments, it can be desirable to further separate the derived recombinant ASFV particles from other proteins.

In some embodiments, the isolation of recombinant ASFV particles may involve size exclusion chromatography or ion chromatography, such as anion exchange, cation exchange, or mixed mode chromatography. In some embodiments, the isolation of recombinant ASFV particles may involve desalting, dialysis, tangential flow filtration, ultrafiltration, or diafiltration, or any combination thereof. In some embodiments, the isolation of recombinant ASFV particles may involve combinations of methods that include, but are not limited to, differential centrifugation, size-based membrane filtration, concentration and/or rate zonal centrifugation. In some embodiments, the isolation of recombinant ASFV particles may involve one or more centrifugation steps.

### Further aspects of the invention

1. A recombinant African swine fever virus (ASFV), characterized in that
   (i) the genome of the recombinant ASFV comprises at least one miRNA target sequence,
   (ii) the miRNA target sequence is found within a gene that is essential for viral replication and
   (iii) the miRNA target sequence is specific for a miRNA which is expressed in a viral target cell.
2. The recombinant ASFV according to aspect 1 wherein the miRNA target site is inserted within the gene that is essential for viral replication in the region which encodes the 3'-UTR of the corresponding mRNA.
3. The recombinant ASFV according to aspects 1 or 2, wherein multiple copies of the miRNA target sequence are inserted in tandem.
4. The recombinant ASFV according to any of aspects 1 to 3 wherein the gene that is essential for viral replication is the G1211R gene.
5. The recombinant ASFV according to any of aspects 1 to 4 wherein the viral target cell is a porcine alveolar macrophage (PAM).
6. The recombinant ASFV according to aspect 5 wherein the miRNA which is expressed in PAM is the miRNA-142-3p.
7. The recombinant ASFV according to aspect 6 wherein the miRNA target sequence comprises the sequence SEQ ID NO:1.
8. The recombinant ASFV according to any aspect 1 to 7, wherein the recombinant ASFV is derived from the hemadsorbing ASFV isolate Arm/07 genotype II.
9. The recombinant ASFV according to aspect 8, wherein the recombinant ASFV genome is derived from the ASFV Arm/07/CBM/c2 strain, having the genome as defined in the GenBank accession number LR812933.1.
10. The recombinant ASFV according to any aspect 1 to 7, wherein the recombinant ASFV is derived from the non-hemadsorbing ASFV isolate NH/P68 genotype I.
11. The recombinant ASFV according to any of aspects 1 to 10, which contains four copies in tandem of the miRNA-142 target sequence at the 3'-UTR of the G1211 R gene.
12. The recombinant ASFV according to any preceding aspect further comprising a reporter gene under the control of a promoter of a late ASFV gene.
13. The recombinant ASFV according to aspect 11, wherein the reporter gene is operably linked to the p72 promoter.
14. A polynucleotide comprising a first, second and third regions, wherein the first region comprises the region of an ASFV gene which encodes the 3'-UTR of the mRNA encoded by said gene, which region is modified by replacing at least part of said region by at least one miRNA target site, wherein the first region is flanked by the second and third regions and wherein said second and third regions are the ASFV genomic regions which naturally flank the first genomic region in the ASFV genome.
15. The polynucleotide according to aspect 14, wherein the ASFV gene is an essential gene for viral replication.
16. The polynucleotide according to aspects 14 or 15, wherein the ASFV gene is selected from the group comprised by the genes G1211R, B646L or 602L.
17. The polynucleotide according to any of aspects 14 to 16, wherein the miRNA target sequence is specific for miR-142-3p.
18. The polynucleotide according to any of aspects 14 to 17, wherein several copies of the miRNA target sequence are arranged in tandem.
19. The polynucleotide according to any of aspects 14 to 18, wherein the polynucleotide contains four copies in tandem of the miRNA-142 target sequence at the 3'-UTR of the G1211R gene.
20. A vector comprising the polynucleotide according to any of aspects 14 to 19.
21. The vector according to aspect 20, further comprising a reporter gene coding for a detectable marker.
22. A host cell comprising the polynucleotide according to any of aspects 14 to 19 or the vector according to aspects 20 or 21.
23. An immunogenic composition or a vaccine composition comprising the recombinant ASFV according to any of aspects 1 to 13, a polynucleotide according to any of aspects 14 to 19 or a vector according to aspects 20 or 21 and a pharmaceutically suitable carrier or excipient.
24. A recombinant ASFV according to any aspect 1 to 13, a polynucleotide according to any of aspects 14 to 19, a vector according to aspects 20 or 21 or a vaccine or immunogenic composition according to aspect 23, for use in the prevention or treatment of a disease caused by the infection by ASFV.
25. A method of inducing a protective immune response to an ASFV infection in an animal, said method comprising administering to said animal a recombinant ASFV according to any aspect 1 to 13, a polynucleotide according to any of aspects 14 to 19, a vector according to aspects 20 or 21 or a vaccine composition according to aspect 23.
26. A method for producing a recombinant African swine fever virus (ASFV) according to any of claims 1 to 10, the method comprising:
   (i) modifying permissive viral target cells by
      - introducing a polynucleotide comprising a first, second and third regions, wherein the first region comprises the region of an ASFV gene essential for viral replication which encodes the 3'-UTR of the mRNA encoded by said gene, which region is modified by replacing at least part of said region by at least one miRNA target site, wherein the first region is flanked by the second and third regions and wherein said second and third regions are the ASFV genomic regions which naturally flank the first genomic region in the ASFV genome, wherein the polynucleotide further comprises a reporter gene between the second and third region,
      - infecting the cells with an ASFV strain in which the gene that is essential for viral replication does not contain a miRNA target site and
      - introducing means capable of creating a double strand DNA break in the genome of said ASFV strain within or at the vicinity of the region encoding the 3'UTR of mRNA encoded by the essential gene for viral replication,
   (ii) maintaining the cells under conditions adequate for the double-strand DNA break in the ASFV genome to take place and to allow homologous recombination between the ASFV genome containing the DNA break and the second and third regions of the polynucleotide thereby resulting in the replacement of the region encoding the 3'UTR of mRNA encoded by the ASFV gene essential for viral replication by the first region of the polynucleotide and the reporter gene, and
   (iii) recovering the recombinant ASFV from the supernatant and/or from the whole cell extract and selecting the ASFV virions which contain the reporter gene.
27. The method of aspect 26, wherein the permissive viral target cell is a mammalian cell line.
28. The method according to aspect 27, wherein the permissive viral target cells are COS cells.
29. The method of any of aspects 26 to 28, wherein the means capable of creating a double strand break in the genome of said ASFV strain within or at the vicinity of the region encoding the 3'UTR of mRNA encoded by the essential gene for viral replication comprise a CRISPR/Cas system.
30. The method according to any of aspects 26 to 29, wherein the reporter gene encodes a fluorescent protein.
31. The method according to aspect 30, wherein the reporter gene is under the control of a constitutive promoter.

The invention is detailed below by means of the following examples which are merely illustrative and by no means limiting the scope of the invention.

### EXAMPLES

### Materials and Methods

### Cell culture, viruses and infections

COS-1 cells (African green monkey kidney, ATCC) grow in Dulbecco's Modified Eagle's Medium (DMEM) supplemented with 2 mM L-glutamine, 100 U/ml gentamicin, non-essential amino acids and 5% fetal bovine serum (FBS) (Invitrogen Life Technologies). Porcine alveolar macrophages (PAM) were prepared by bronchoalveolar lavage and grow in DMEM supplemented with 2 mM L-glutamine, 100 U/ml gentamicin, non-essential amino acids and 10% porcine serum. Cells were grown at 37°C in a 7% CO2 atmosphere saturated with water vapor.

The ASFV isolate Arm/07/CBM/c2 (Pérez-Núñez, et al., 2020) was propagated in PAM by infection at a multiplicity of infection (MOI) of 0.5 in DMEM 10% porcine serum. At 72 h post infection (hpi), infected cells were recovered and centrifuged at 3,000 rpm for 15 min. Pellet was discarded and the virus-containing supernatant clarified at 14,000 rpm for 6 h at 4°C and the purified infectious virus resuspended in medium and stored at -80°C. ASFV viral adsorption to cells was performed by shaking at 37°C for 90 min and then infection at 37°C until the hpi indicated.

### Fluorescence Activated Cell Sorting (FACS)

COS-1 cells were transfected with the following vectors (kindly provided by Mount Sinai, ICAHN School of Medicine) pEGFP, pEGFP-C1-miR-142-3px4 (EGFP_142), pMi142 and pMi9, and then Mock-infected or infected with Arm/07/CBM/c2. After the indicated times, cells were detached with trypsin-EDTA, fixed with 2% paraformaldehyde for 30 min at 4 °C and then permeabilized with PBS-Staining buffer (0.5% BSA in PBS) 0.2% saponin for 15 min at RT. Detection of infected cells was performed by incubation with anti-viral p72 monoclonal antibody (17LD3, CBMSO) and/or anti-viral p32 antibody (5-SC1 KSI/CBMSO) (diluted 1:100 in PBS-Staining buffer 0.2% saponin) for 20 min at 4°C, followed by incubation with an anti-mouse Alexa Fluor-647 (diluted 1:500 in PBS-Staining buffer, 0.2% saponin) in the same conditions. Finally, cells were analyzed in a FACSCanto A (Becton Dickinson) by FlowJo 7.2.2 software.

### Cloning of vectors for the generation of Arm07/c2/G1211R-miR142-GFP by CRISPR-Cas9 technology

We adapted the CRISPR-Cas9 technology to substitute the 3'UTR of the G1211 R gene by a construction consisting of miR142 target in tandem (kindly provided by Mount Sinai, ICAHN School of Medicine), together with GFP gene under the viral p72 promoter. For that, two vectors were used: (i) one derived from pSpCas9(BB)-2A-Puro (PX459), containing the Cas9 nuclease of Streptococcus pyogenes and a gene conferring resistance to Puromycin in which Nuclear Localization sequence (NLS) has been deleted (pSpCas9(BB)ΔNLS-2A-Puro); and (ii) a pcDNA3.1-derived vector containing the flanking sequences of the target sequence (3'UTR of the G1211 R gene), surrounding by the miR142 target miR-142-3p in 4x tandem, the marker gene EGFP derived from the pEGFP-E3 vector under ASFV p72 promoter, together with the CP123L gene with its transcription terminal signal.

For the pSpCas9(BB)ΔNLS-2A-Puro-derived vectors, we cloned specific gRNAs to disrupt the CP123L gene, which is located downstream G1211R gene. For the design of the gRNAs we used Protospacer, based on the sequence of the Arm/07/CBM/c2 ASFV strain (LR812933.1). The designed gRNA sequences were as follow: ATAACCAGTGTCCCAGTCGAGGG ("gRNA_CP123L-0") and GCTTTTTTGGCGAATCATAAGGG ("gRNA_CP123L-1"). Each of these gRNAs were cloned into the pSpCas9(BB)ΔNLS-2A-Puro vector following the procedure described in (Ran, Hsu et al. 2013), generating the vectors pSpCas9(BB)ΔNLS-2A-Puro_CP123L-gRNA-0 and pSpCas9(BB)ΔNLS-2A-Puro_CP123L-gRNA-1.

For the cloning of the donor vector pFLmiR142(G1211R)-p72GFP-CP123 we used InFusion technology, generating intermediate vectors. Firstly, we generated the pcDNA-CMVp72-EGFP using pcDNA3.1 as a backbone and substituting the MCS for the viral promoter p72 followed by EGFP, from the vector pFLΔEP402R-p72EGFP (previously generated in CBMSO). Then, we cloned the miR-142-3p4x from the pEGFP-C1-miR-142-3px4, upstream of p72-EGFP, generating the vector pmiR142-p72EGFP. In parallel we generated the pFL-CP123L vector, using pcDNA3.1 as a backbone in which we cloned the CP123L gene (117,554-117,922) and its flanking regions (500pb upstream and 500pb downstream) from Arm/07/CBM/C2 ASFV strain. We then cloned the CP123L together with 123 bp downstream (containing its transcriptional termination signal, "7T") from pFL-CP123L within pmiR142-p72EGF, generating the pmiR-p72EGFP-CP123L-7T. Finally, we substituted the CP123L gene from the pFL-CP123L, by the miR-142-3p4x-p72-EGFP-CP123L-7T construction from the pmiR-p72EGFP-CP123L-7T, generating the pFLmiR142(G1211R)-p72GFP-CP123L.. The oligo probes used for those cloning generated by InFusion technology is displayed in Table 2.

**Table 2. Oligo probes used for the generation of the indicated vectors**

| Vector | Oligos |
|---|---|
| pcDNA-CMV-p72EGFP | 5'-GAGACCCAAGCTGGCTAGC-3' and 5'-AACGCGTATATCTGGCCCG-3' |
| | 5'- CCAGATATACGCGTTGACATTGATTATTGACTAGTTATTAATAGTAATCAATTACG G-3' AND 5'-GCCAGCTTGGGTCTCTTACTTGTACAGCTCGTCCATGC-3' |
| pmiR142-p72EGFP | 5'-TATTTAATAAAAACAATAAATTATTTTTATAACATTATATAGGTCGCCA-3' and 5'- AACGCGTATATCTGGCCCG-3' |
| | 5'-CCAGATATACGCGTTGTCGACTCCATAAAGTAGGAAACACTACACG-3' and 5'- TGTTTTTATTAAATATAAGATACATTGATGAGTTTGGACAAACC-3' |
| pFL-CP123L | 5'-CTTCTGAGGCGGAAAGAACCA-3' and 5'-AACGCGTATATCTGGCCCG-3' |
| | 5'-CCAGATATACGCGTTAAACCATTCTATGAAAAAAATGGTTGAACAGC-3' and 5'- TTTCCGCCTCAGAAGTCATATACAGAAAATAGTGTGCTGACCTATATCAC-3' |
| pmiR142-p72EGFP-CP123L-7T | 5'-AATTCTGTGGAATGTGTGTCAGTTAGG-3' and 5'- GGGGATACCCCCTAGAGCC-3' |
| | 5'- CTAGGGGGTATCCCCAAAAAAACATCTGCAAGCTTTTTTGGAT-3' and 5'- ACATTCCACAGAATTATGCCCTCGACTGGGAC-3' |
| pFLmiR142(G 1211R)-p72EGFP-CP123L | 5'- TTTGTTATATAAGTAACGTTTTTTTTTATTTTTTATAATTCGAATGAAGA-3' and 5'-TTAGGAAATAAAGTCGTAGCAAGATGGTTT-3' |
| | 5'-GACTTTATTTCCTAAGTCGACTCCATAAAGTAGGAAACACTACACG-3' and 5'- TACTTATATAACAAAATGCCCTCGACTGGGACAC-3' |

### Generation of recombinant virus Arm07/c2/G1211R-miR142-GFP by CRISPR-Cas9 technology

COS-1 cells grown as indicated above, were seeded in a 6-well plate at 90% confluence and individually wells were co-transfected with 2µg per vector of either pSpCas9(BB)ΔNLS-2A-Puro gRNA-0 and pFLmiR142(G1211R)-p72GFP-CP123L or pSpCas9(BB)ΔNLS-2A-Puro gRNA-1 and pFLmiR142(G1211R)-p72GFP-CP123L with FuGene HD (Promega). 24h post transfection, puromycin (Sigma) was added to the media of transfected and no transfected control cells (1µg/ ml). After 48h, selected cells were infected at two different MOI (1 and 0.5) with Arm/07/CBM/c2 ASFV strain. 5-7 days post infection (dpi) cell and medium was collected and conserved at -80°C.

### Isolation of recombinant viruses from wild-type viruses by plaque isolation

After three freeze/thaw cycles of the transfected/infected cells, supernatant-containing virus was used to infect COS-1 cells. After 1h 30 minutes of viral adsorption, inoculum is removed and Carboxymethylcellulose (CMC) was added. After 4-7 dpi green viral plaques were identified by GFP present in the recombinant viruses) at the fluorescent microscopy and further collected by sterile tips in 40µl of DMEM and conserved at -80 °C. After three freeze / thaw cycles, extracted virus was used to infect new COS-1 cells with the same procedure explained above. This plaque isolation was repeated at least three times in order to isolate the recombinant from the wild type virus.

During the isolation procedure, the presence of putative wild type contaminant viruses was checked by PCR. For that, 10µl of the isolated plaque is digested with proteinase K (Sigma) in 1.5mM MgCl₂, 50mM KCl, 0.45% Tween20, 0.45% NP40 and 10mM TrisHCl pH8.3 buffer, incubated 30 minutes at 45 °C and 15 minutes at 95 °C. The digested isolated plaque is used as a DNA template for PCR to further detect the presence of recombinant and parental virus. The oligos used corresponding to each recombinant virus are listed in Table 3.

**Table 3. Oligos used to determine the presence of wild-type and recombinant Arm/07/CBM/c2-G1211RmiR 142-GFP**

| | |
|---|---|
| Identification Wild-type Arm/07/CBM/c2 virus | 5'- GCA GCT GAT TAC GGA AGA AAA AAA AAT TG-3' and 5'-TAC TTA TAT AAC AAA ATG CCC TCG ACT GGG ACA C-3' |
| Identification recombinant Arm/07/CBM/c2-G1211R-miR142-GFP virus | 5'- GCA GCT GAT TAC GGA AGA AAA AAA AAT TG-3' and 5'-GAA CTT GTG GCC GTT TAC GT-3' |

### PCR and Sanger Sequencing

Conventional PCR for validation of miR-142-3p4x insertion within the 3'UTR of the G1211R gene at the recombinant virus was performed with Phusion High-Fidelity PCR Master Mix with HF Buffer (Thermo Scientific, USA) and the following primers: 5'-GCA GCT GAT TAC GGA AGA AAA AAA AAT TG-3' and 5'-GAA CTT GTG GCC GTT TAC GT-3'. Corresponding band was purified from a 1% agarose gel with Speedtools PCR Clean-Up kit (Biotools, Madrid, Spain). The purified products were subsequently sequenced by the Sanger method (Macrogen, Seoul, Korea).

### Quantitative PCR (qPCR)

COS-1 cells were seeded (2·106 cells) and Mock-infected or infected with Arm/07/CBM/c2-G1211R-miR142-GFP, from either COS-1 cells or PAM. At 72 hpi total DNA was harvested from cells using a Speedtools DNA extraction kit (Biotools). DNA was analyzed by qPCR using an ABI PRISM 7900HT SDS real-time PCR detection system (Applied Biosystems) with SYBR green master mix (Promega). Gene expression levels were normalized to the housekeeping gene (18S rRNA), and these values were then normalized to the mock-treated sample. The primers used were 5'-GGCCCGAGGTTATCTAGAGTC-3' and 5'-TCAAAACCAACCCGGTCA-3' for porcine 18S rRNA detection, 5'- AAAAATGATAATGAAACCAATGAATG -3' and 5'-ATGAGGGCTCTTGCTCAAAC-3' for ASFV CP204L (p32) detection, 5'-GGTTTCATCCATTCAATTAGGC-3' and 5'-CGCTCTCTGTTGGCCTTAAA-3' for ASFV B602L detection, and 5'-TAGCCGACGTGGCCTATTAC-3' and 5'-GCACCAGAAGGTCCTGACA-3' for ASFV G1211R detection.

### Western blot analysis

PAM and COS-1 cells mock or ASFV infected were collected at the specified times post-infection, washed with PBS, and lysed with radioimmunoprecipitation assay (RIPA) buffer supplemented with protease and phosphatase inhibitors (Roche) for 30 min at 4 °C, sonicated, and centrifuged at 13,000 rpm for 10 min at 4°C. The supernatants were collected, and equal amounts of protein per sample were used. Samples were resolved by sodium dodecyl sulfate polyacrylamide gel electrophoresis (SDS-PAGE) and transferred to Immobilon-P membranes (Millipore). The membranes were incubated with the following specific primary antibodies anti-1262 (serum from ASFV-infected pig) antibody (1/1,000), anti-viral CD2v antibody (1/4,000) and anti-viral p32 antibody (1/5,000), diluted in Tris-buffered saline (TBS) supplemented with 1% milk. Membranes were washed three times with TBS and exposed 1 h to specific peroxidase-conjugated secondary antibodies: anti-rabbit and anti-mouse immunoglobulin G coupled to peroxidase (1/5,000 and 1/2,000, respectively) from Amersham Biosciences and anti-m-IgGκ secondary antibody (1/1,000) from Santa Cruz Biotechnology. Chemiluminescence detection was performed using ECL Prime (Amersham Biosciences).

### EXAMPLE 1

### ASFV infection does not prevent miR142 degradation activity

Firstly, and in order to investigate if miR142 activity might be impaired during ASFV infection, we used the vectors EGFP_142 (EGFP containing target for miR142) and pMi-142 (coding for miR142) together with control vectors EGFP (with no target for miR142) and pMi-9 (coding for miR9). We evaluated the degree of EGFP_142 degradation in cells co-transfected with pMi-142 and whether ASFV infection interfered with this degradation, in presence or not of DNA replication inhibitor AraC. AraC blocks viral DNA replication allowing only early viral protein expression, since late viral protein expression does not occur.

We transfected COS cells with the follow combinations: EGFPc+miR9, EGFP_142+miR9, EGFPc+miR142, EGFP_142+miR142, and after 24h cells were mock-infected or Arm/07/CBM/c2-infected (MOI= 0.2), which were treated or not with AraC. At 48 hpt cells were collected and stained with anti-p32 (early ASFV protein) and subject to FACS analysis. We stained infected cells, treated or not with AraC, with anti-p72 (late ASFV protein) as a control of AraC function. As shown in Fig. 1A, degradation of EGFP occurred when cells were co-transfected with EGFP_142+miR142, but not in other combinations, indicating the specificity of the miR142 in degrading subtracts carrying the specific target. Importantly, this specific degradation was also observed in ASFV-infected cells, both in the presence and in the absence of AraC. ASFV infection was quantified by the expression of p32, and the effect of AraC in ASFV infection by the expression of p72 (Fig. 1B).

To further discard the ASFV infection impairment over the miR142 specific degradation process, we analyzed the effect of simultaneous ASFV infection in transfected cells to rule out an ASFV-dependent early mechanism. For that, we first proceed with viral adsorption (1h30) with Arm/07/CBM/c2 (MOI= 0.2) and then transfect both infected and mock-infected cells. At 16 hpi cells were collected, stained with anti-p32 and subject to FACS analysis. As it can be observed in Fig. 2A, we observed the specific degradation of EGFP_142 when co-transfected with miR142, in both mock-infected and Arm/07/CBM/c2-infected cells, similarly to what we observed in Fig. 1A. Fig 2B showed that infection over the cells transfected with the different combinations were similar.

Altogether, these data indicate that ASFV infection does not prevent miR142-mediated degradation, thus allowing the use of this system for the next step for vaccine development.

### EXAMPLE 2

### Generation of recombinant Arm07/CBM/c2-G1211R-miR142-GFP by CRISPR-Cas9

Transfected and Arm/07/CBM/c2-infected COS-1 cells, as explained in Methods, were used to produce clones of the recombinant Arm07/CBM/c2-G1211 R-miR142-GFP (Fig. 3). The resultant individual plates (six) were grown in 6-well plates of COS cells until full cytopathic effect was observed (more than 50% cells were GFP+) and then were recovered and subjected to three further freeze/thaw cycles; the resultant extracts were called "pre-Master stocks". 100µl from each was used to infect another M6 well plate for until complete cytopathic effect was observed (around 80% cells were GFP+). 10µl of these samples were digested with proteinase K and the resultant product used as a template for PCR amplification using the following specific probes indicated in Table 2 for wild-type amplicon or for GFP-recombinant virus amplification.

The combination of oligos displayed in Table 2 should generate a band of 723 bp in the wild-type virus genome, and non-amplification of the recombinant virus genome, and a band of 923 bp in the recombinant virus genome, and non-amplification in the wild-type virus genome.

The results from PCRs showed that all recovered clones consisted of the recombinant virus Arm-G1211R-miR, and all of them A2 (1 and 2), B1 (1 and 2) and C (1 and 2) appeared free of Arm-WT contaminants (Fig. 4A). In addition, we have investigated if these clones contained off-target deletions, which could appear both at the left arm of the genome (encompassing the members of MGF) and specific regions of the central region (EP153R and EP402R genes). To assess this, we have checked the presence or deletion of the genes MGF-110-13L and MGF-360-8L (Fig. 4 B), MGF-360-4L (Fig. 4 C) and EP402R (Fig. 4 D) using specific oligonucleotides. As observed in the Fig. 4, none of the clones showed deletion in these genes except for clone C.2, concerning MGF-110-13L gene (Fig. 4 B).

### EXAMPLE 3

### Sanger sequencing confirms the correct miR142 target insertion in the 3'UTR of the G1211R gene

In order to verify the insertion of the tandem repetitions of miR-142 target in the 3'UTR of the G1211R gene, we amplified by PCR this region with the oligos indicated in Table 2 using a Phusion High-Fidelity polymerase as indicated in Materials & Methods, of the clones A2.1 and B1.1. The results showed that the miR142 target was correctly inserted in the 3' UTR of the G1211R gene (Fig. 5), in the selected clones A2.1 and B1.1, which displayed the correct insertion and no off-target deletions/mutations in the studied genes.

### EXAMPLE 4

### The recombinant Arm07/CBM/c2-G1211R-miR142-GFP displays impaired ability to replicate and to generate infective viral particles in PAM

In order to investigate the behavior of the recombinant virus and the consequences of the presence of the miR142 target, we tested the global viral protein expression and/or viral production in PAM by using an experimental approach based on the selective growing of the recombinant virus only in cells which do not express the miRNA142-3p.

Following this approach, Arm07/CBM/c2-G1211R-miR142 clones A2.1 or B.1, or Arm07/CBM7C2 wild-type, were used to infect PAM cells at a MOI= 0.005. At 72 hpi cells were collected and one fraction was separate and conserved at -80 °C to be used in additional experiments, and the rest of the sample was processed for Western blot, to assess the expression of viral proteins. 100µl from the 1ml-fraction 1st passage were used to infect new PAM and the same protocol was repeated to generate the 2nd passage, and again to generate the final 3rd passage of viruses in PAM.

Extracts of infected PAM from 1st and 3rd passage were subjected to Western blot in order to analyze viral protein expression. As shown in Fig. 6, clones A2.1 and B1.1 of Arm07/CBM/c2-G12114R-miR142-GFP are able to produce some degree of viral proteins expression pattern (mainly ASFV early proteins) in PAM infected from the first passage (produced in COS). However, in PAM infected with the third passage almost no viral protein expression was found. In control PAM cells infected with Arm07/CBM/c2 WT, a full viral protein expression pattern (early and late) was found from both first and third passages, (substantially increasing in the third passage). Therefore, recombinant virus produced in COS cells is able to infect and produce viral proteins once in PAM, but the virus produced in those PAM is not any longer able to produce viral proteins in the next round in PAM. These results indicate that Arm07/CBM/c2-G12114R-miR142-GFP is not able to produce new viral infectious progeny in PAM, suggesting that likewise it would produce an abortive infection in the animals.

It is noteworthy that in Arm07/CBM/c2-G12114R-miR142-GFP-infected PAM no bands corresponding to late ASFV proteins (p72, p17, p12 or CD2v) were detected, compared to the pattern of bands found in Arm07/CBM/c2-WT-infected PAM. However, the early viral protein p32 was similarly expressed in PAM infected with recombinant or WT virus, in the first passage. This may indicate a specific role of G1211R protein in the expression of late viral proteins, and would not be surprising since expression of late viral proteins, but not always early proteins, depends on viral DNA replication and G1211R functions as a viral DNA polymerase.

Moreover, we quantified the relative amount of viral DNA found in PAM infected with either Arm07/CBM/c2 WT or G12114R-miR142 mutants in the first, second and third passage. For that, we analyze specific sequences corresponding to viral ORFs B602L, CP204L and G1211R by qPCR. As shown in Fig. 7, relative viral DNA detected in infected PAM collected from first passage decreased from about 2 log in Arm07/CBM/c2-G1211R-miR142-GFP-infected cells compared to WT-infected cells, indicating that viral DNA replication is impaired in PAM cells infected with recombinant virus. Even more important, viral DNA from G1211R-miR142-GFP-infected PAM further decreased over 3 log in 3rd passage compared to 1st passage, whereas viral DNA from Arm07/CBM/c2-WT-infected PAM still increased one log, indicating enriched viral replication (Fig. 8). These results indicate that viral production of recombinant virus is fully impaired in PAM, in agreement with viral protein expression data. Furthermore, and from the first passage, we detected a progressive decrease in viral DNA replication over the passages, indicating that no new infective particles are being produced.

Finally, extracts collected from PAM infected with either Arm07/CBM/c2 WT or G1211R-miR142 clones A2.1 or B1.1 (1st to 3rd passage) were used to infect COS cells. COS cells are permissive for recombinant G1211R-miR142 viruses, and hence, they were used to check productive viral particles. As shown in Fig. 8, extracts collected from Arm/07/CBM/c2 WT-infected PAM, from all passages, were able to productively infected COS cells and produce viral proteins such as p72, p17 and p12, in similar amount between the different passages. However, extracts from PAM infected both with Arm/07/CBM/c2 G1211R-miR142-GFP A2.1 (Fig. 8A) and B1.1 clones (Fig. 8B), barely expressed viral proteins in COS cells, suggesting that very low or no virus was present in the extracts from passages in PAM.

### Summary

In conclusion, Arm07/CBM/c2-G1211R-miR142-GFP recombinant virus, which contains the target sequence of miR142 at the 3'UTR of the ASFV DNA polymerase, is fully able to replicate and to grow in COS cells. In contrast, our results shown that in PAM, the recombinant virus is impaired to develop the ASFV replicative cycle, suggesting that PAM miR142 efficiently targets the G1211R-miR142 construction at the mRNA of the viral polymerase. As a consequence, both viral DNA replication and most

## Claims

1. A recombinant African swine fever virus (ASFV), **characterized in that**
(i) the genome of the recombinant ASFV comprises at least one miRNA target sequence,
(ii) the miRNA target sequence is found within a gene that is essential for viral replication and
(iii) the miRNA target sequence is specific for a miRNA which is expressed in a viral target cell.

2. The recombinant ASFV according to claim 1 wherein the miRNA target sequence is inserted within the gene that is essential for viral replication in the region which encodes the 3'-UTR of the corresponding mRNA.

3. The recombinant ASFV according to claims 1 or 2, wherein multiple copies of the miRNA target sequence are inserted in tandem.

4. The recombinant ASFV according to any of claims 1 to 3 wherein the gene that is essential for viral replication is the G1211R gene.

5. The recombinant ASFV according to any of claims 1 to 4 wherein the viral target cell is a porcine alveolar macrophage (PAM).

6. The recombinant ASFV according to claim 5 wherein the miRNA which is expressed in PAM is the miRNA-142-3p.

7. The recombinant ASFV according to claim 6 wherein the miRNA target sequence comprises the sequence SEQ ID NO:1.

8. The recombinant ASFV according to any of claims 1 to 7, wherein the recombinant ASFV is derived from the hemadsorbing ASFV isolate Arm/07 genotype II.

9. The recombinant ASFV according to any of claims 1 to 7, wherein the recombinant ASFV genome is derived from one of the group consisting of the ASFV Arm/07/CBM/c2 strain, having the genome as defined in the GenBank accession number LR812933.1, and the non-hemadsorbing ASFV isolate NH/P68 genotype I.

10. The recombinant ASFV according to any of claims 1 to 9, which contains four copies in tandem of the miRNA-142 target sequence at the 3'-UTR of the G1211R gene.

11. A polynucleotide comprising a first, second and third regions, wherein the first region comprises the region of an ASFV gene which encodes the 3'-UTR of the mRNA encoded by said gene, which region is modified by replacing at least part of said region by at least one miRNA target site, wherein the first region is flanked by the second and third regions and wherein said second and third regions are the ASFV genomic regions which naturally flank the first genomic region in the ASFV genome.

12. A host cell comprising the polynucleotide according to claim 11 or a vector comprising the polynucleotide according to claim 11.

13. An immunogenic composition or a vaccine composition comprising the recombinant ASFV according to any of claims 1 to 10, a polynucleotide according to claim 11 or a vector according to claim 12 and a pharmaceutically suitable carrier or excipient.

14. A recombinant ASFV according to any claim 1 to 10, a polynucleotide according to claim 11, a vector according to claim 12 or a vaccine or immunogenic composition according to claim 13, for use in the prevention or treatment of a disease caused by the infection by ASFV.

15. A method for producing a recombinant African swine fever virus (ASFV) according to any of claims 1 to 10, the method comprising:
(i) modifying permissive viral target cells by
- introducing a polynucleotide comprising a first, second and third regions, wherein the first region comprises the region of an ASFV gene essential for viral replication which encodes the 3'-UTR of the mRNA encoded by said gene, which region is modified by replacing at least part of said region by at least one miRNA target site, wherein the first region is flanked by the second and third regions and wherein said second and third regions are the ASFV genomic regions which naturally flank the first genomic region in the ASFV genome, wherein the polynucleotide further comprises a reporter gene between the second and third region,
- infecting the cells with an ASFV strain in which the gene that is essential for viral replication does not contain a miRNA target site, and
- introducing means capable of creating a double strand DNA break in the genome of said ASFV strain within or at the vicinity of the region encoding the 3'UTR of mRNA encoded by the essential gene for viral replication,
(ii) maintaining the cells under conditions adequate for the double-strand DNA break in the ASFV genome to take place and to allow homologous recombination between the ASFV genome containing the DNA break and the second and third regions of the polynucleotide thereby resulting in the replacement of the region encoding the 3'UTR of mRNA encoded by the ASFV gene essential for viral replication by the first region of the polynucleotide and the reporter gene, and
(iii) recovering the recombinant ASFV from the supernatant and/or from the whole cell extract and selecting the ASFV virions which contain the reporter gene.
